(19) Europäisches Patentamt
European Patent Office
Office européen des brevets

(11) **EP 3 078 978 B1**

(12) **EUROPÄISCHE PATENTSCHRIFT**

(45) Veröffentlichungstag und Bekanntmachung des Hinweises auf die Patenterteilung:
**20.10.2021 Patentblatt 2021/42**

(51) Int Cl.:
*G01R 33/3875* (2006.01) *G01R 33/54* (2006.01)

(21) Anmeldenummer: **16157046.0**

(22) Anmeldetag: **24.02.2016**

(54) **VERFAHREN ZUR MAGNETRESONANZ-BILDGEBUNG**

METHOD OF MAGNETIC RESONANCE IMAGING

PROCEDE D'IMAGERIE PAR RESONANCE MAGNETIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.03.2015 DE 102015204953**

(43) Veröffentlichungstag der Anmeldung:
**12.10.2016 Patentblatt 2016/41**

(73) Patentinhaber: **Siemens Healthcare GmbH 91052 Erlangen (DE)**

(72) Erfinder: **Stemmer, Alto 91054 Erlangen (DE)**

(56) Entgegenhaltungen:
**EP-A2- 1 845 387     US-A1- 2008 088 307**

- **SAIKAT SENGUPTA ET AL: "Dynamic B0 shimming at 7 T", MAGNETIC RESONANCE IMAGING, ELSEVIER SCIENCE, TARRYTOWN, NY, US, Bd. 29, Nr. 4, 2. Januar 2011 (2011-01-02), Seiten 483-496, XP028190842, ISSN: 0730-725X, DOI: 10.1016/J.MRI.2011.01.002 [gefunden am 2011-02-03]**
- **GRAAF DE R A ET AL: "DYNAMIC SHIM UPDATING (DSU) FOR MULTISLICE SIGNAL ACQUISITION", MAGNETIC RESONANCE IN MEDICINE, JOHN WILEY & SONS, INC, US, Bd. 49, Nr. 3, 1. März 2003 (2003-03-01), Seiten 409-416, XP001144372, ISSN: 0740-3194, DOI: 10.1002/MRM.10404**

EP 3 078 978 B1

**Beschreibung**

Die Erfindung betrifft ein Verfahren zur Magnetresonanz-Bildgebung, ein Magnetresonanzgerät und ein Computerprogrammprodukt.

In einem Magnetresonanzgerät, auch Magnetresonanztomographiesystem genannt, wird üblicherweise der zu untersuchende Körper einer Untersuchungsperson, insbesondere eines Patienten, mit Hilfe eines Hauptmagneten einem relativ hohen Hauptmagnetfeld (B0-Feld), beispielsweise von 1,5 oder 3 oder 7 Tesla, ausgesetzt. Zusätzlich werden mit Hilfe einer Gradientenspuleneinheit Gradientenpulse ausgespielt. Über eine Hochfrequenzantenneneinheit werden dann mittels geeigneter Antenneneinrichtungen hochfrequente Hochfrequenz-Pulse, beispielsweise Anregungspulse, ausgesendet, was dazu führt, dass die Kernspins bestimmter, durch diese Hochfrequenz-Pulse resonant angeregter Atome um einen definierten Flipwinkel gegenüber den Magnetfeldlinien des Hauptmagnetfelds verkippt werden. Bei der Relaxation der Kernspins werden Hochfrequenz-Signale, so genannte Magnetresonanz-Signale, abgestrahlt, die mittels geeigneter Hochfrequenzantennen empfangen und dann weiterverarbeitet werden. Aus den so akquirierten Rohdaten können schließlich die gewünschten Bilddaten rekonstruiert werden.

Für eine bestimmte Messung ist daher eine bestimmte Magnetresonanz-Sequenz, auch Pulssequenz genannt, auszusenden, welche aus einer Folge von Hochfrequenz-Pulsen, beispielsweise Anregungspulsen und Refokussierungspulsen, sowie passend dazu koordiniert auszusendenden Gradientenpulsen in verschiedenen Gradientenachsen entlang verschiedener Raumrichtungen besteht. Zeitlich passend hierzu werden Auslesefenster gesetzt, welche die Zeiträume vorgeben, in denen die induzierten Magnetresonanz-Signale erfasst werden.

Bei der Magnetresonanz-Bildgebung mittels eines Magnetresonanzgeräts ist die Homogenität eines Hauptmagnetfelds in einem Untersuchungsvolumen von großer Bedeutung. Bereits bei kleinen Abweichungen der Homogenität kann es zu großen Abweichungen in einer Frequenzverteilung der Kernspins kommen, so dass qualitativ minderwertige Magnetresonanz-Bilddaten aufgenommen werden. Beispielsweise stellen Magnetresonanz-Aufnahmemethoden wie eine echoplanare Bildgebung oder eine spirale Bildgebung hohe Anforderungen an die Homogenität des Hauptmagnetfelds.

Eine Inhomogenitätsquelle stellt das aufzunehmende Untersuchungsobjekt an sich dar. Wird beispielsweise eine zu untersuchende Person in das Magnetresonanzgerät eingebracht, so stört die Materie des Körpers die Homogenität des Hauptmagnetfelds.

Um diesem Problem zu begegnen, ist es bekannt, eine justierbare Shimeinheit zu verwenden. Mittels dieser Shimeinheit kann individuell für verschiedene Untersuchungsobjekte ein Shimvorgang, ein sogenanntes "in vivo shimming", durchgeführt werden. Bei diesem Shimvorgang wird üblicherweise zunächst ein lokales B0-Feld in einem Untersuchungsbereich gemessen, wobei eine B0-Feldkarte, auch B0-Feldkarte oder B0-map genannt, erstellt wird. Anhand der B0-Feldkarte können konstante Shimströme (DC-Offset Shimströme), welche durch Gradientenspulen des Magnetresonanzgeräts fließen ermittelt werden. Weiterhin können anhand der B0-Feldkarte Ströme für spezielle Shimkanäle höherer Ordnung berechnet werden, die lokale Feldverzerrungen besonders vorteilhaft kompensieren können. Nach Einstellung dieser Ströme wird in der Regel in einer Frequenzjustage eine Resonanzfrequenz für gewünschte spektrale Komponente eines untersuchten Gewebes, insbesondere von an Wasser gebundenen Protonen, ermittelt werden. Die DC-Offset Shimströme und/oder die Ströme für die Shimkanäle höherer Ordnung und/oder die Resonanzfrequenz können dann einen Shimparametersatz bilden, welcher für das Erfassen von Magnetresonanz-Bilddaten eingesetzt wird.

Es ist bekannt, dass eine Kompensation von lokalen Feldverzerrungen umso vollständiger funktioniert, je kleiner ein Volumen ist in dem das Hauptmagnetfeld homogenisiert werden soll. Es kann deshalb Sinn machen verschiedene Shimparametersätze für mehrere Teilbereiche des Untersuchungsbereichs separat zu ermitteln und die Shimeinheit anhand der verschiedenen Shimparametersätze für das Erfassen von Magnetresonanz-Bilddaten aus den mehreren Teilbereichen unterschiedlich einzustellen. Dabei kann ein rasches Umschalten der Shimströme und der Resonanzfrequenz während einer Laufzeit der Magnetresonanz-Sequenz zum Erfassen der Magnetresonanz-Bilddaten auf einer sequenzabhängigen Zeitskala von wenigen Millisekunden bis einigen Sekunden nötig sein. Deshalb spricht man üblicherweise bei dieser Art eines Shimvorgangs auch von einem dynamischen Shimmen (dynamic shimming).

Dynamisches Shimmen wird beispielsweise in der Schrift Blamire et al., "Dynamic Shim Updating: A New Approach Towards Optimized Whole Brain Shimming", 1996, MRM, 36, 159-165 und in der Schrift Morrell et al., "Dynamic Shimming for Multi-Slice Magnetic Resonance Imaging", 1997, MRM, 38, 477-483 beschrieben. Weiterhin sind Methoden zum dynamischen Shimmen aus den Schriften von Sengupta et al., "Dynamic B0 shimming at 7T", Magnetic Resonance Imaging, 29, 483-496 (2011) und von de Graaf et al., "Dynamic Shim Updating (DSU) for Multislice Signal Acquisition", Magnetic Resonance in Medicine, 49, 409-416 (2003) bekannt. Eine Methode zur Optimierung von Shimparametern ist aus der US 2008/0088307 A1 bekannt.

Die Genauigkeit mit der lokale Inhomogenitäten des Hauptmagnetfelds kompensiert werden hängt insbesondere von der Zahl und Ordnung von vorhandenen und nutzbaren Shimkanälen ab. Bei modernen MR-Anlagen werden lineare Shimterme, beispielsweise in den drei Raumrichtungen, in der Regel über DC-Offset Shimströme der drei Gradientenspulen erzeugt. Wegen des linearen Feldverlaufs entlang der Gradientenrichtungen kann man die mit den DC-Offset Shimströmen belegten Gradientenspulen dann Shimkanäle erster Ordnung nennen. Weiterhin können Magnetresonanzgeräte über dedizierte Shimspulen verfügen. Diese dedizierten Shimspulen können dann Shimkanäle höherer Ordnung, beispielsweise zweiter Ordnung, bilden. Solche Shimkanäle sind häufig so konstruiert, dass die von ihnen erzeugten Kompensationsfelder durch Kugelflächenfunktionen beschrieben werden können. So können diese Shimkanäle Kompensationsfelder höherer Ordnung erzeugen. Beispielsweise umfassen typische Shimkanäle 2. Ordnung fünf Shimspulen.

Die Gradientenspulen und zugehörige Gradientenverstärker sind typischerweise so ausgelegt, dass die Gradientenfelder auf einer Zeitskala von wenigen Mikrosekunden, beispielsweise von ungefähr zehn Mikrosekunden, variiert werden können. Dies ist für das dynamische Shimmen üblicherweise ausreichend. Das Feld von Shimkanälen höherer Ordnung stellt sich dagegen typischerweise erst nach einer Einschwingzeit (settle time) in einer Größenordnung einer Sekunde ein. Dies ist für das dynamische Shimmen häufig nicht hinreichend. Die Ursache dafür kann bauartbedingt sein, da ein schnelleres Einstellen dieser Shimkanäle stärkere Verstärker und/oder eventuell Drähte mit geringem Widerstand erfordert. Dies kann die Kosten eines Magnetresonanzgeräts erhöhen. Eventuell kann auch ein Platzbedarf der Shimspulen der Shimkanäle höherer Ordnung limitierend sein.

Aus diesen Gründen schaltet man bei einem dynamischen Shimvorgang häufig nur die Shimkanäle erster Ordnung, nämlich üblicherweise die Gradientenspulen, und/oder eine sich aus einer Frequenzjustage ergebende Mittenfrequenz (RF center frequency) dynamisch. So variieren während der Laufzeit der Magnetresonanz-Sequenz typischerweise nur die Ströme in den Shimkanälen erster Ordnung. In den Shimkanälen höherer Ordnung stellt man üblicherweise einmalig vor der Messung einen Strom ein. Dieser Strom soll dann konstant über die Messung gehalten werden. Der konstante DC-Offset Shimstrom kann einem gerätespezifischen Tuneup Wert entsprechen oder in einer vorab durchgeführten für das Untersuchungsobjekt spezifischen Justage ermittelt werden.

Der Erfindung liegt die Aufgabe zu Grunde, eine verbesserte Einstellung von zumindest einem Shimkanal für die Magnetresonanz-Bildgebung zu ermöglichen. Die Aufgabe wird durch die Merkmale der unabhängigen Ansprüche gelöst. Vorteilhafte Ausgestaltungen sind in den Unteransprüchen beschrieben.

Das erfindungsgemäße Verfahren zur Magnetresonanz-Bildgebung in einem Untersuchungsbereich eines Untersuchungsobjekts mittels eines Magnetresonanzgeräts, welches eine Shimeinheit umfasst, wobei die Shimeinheit eine erste Shimkanalmenge mit zumindest einem ersten Shimkanal und eine zweite Shimkanalmenge mit zumindest einem zweiten Shimkanal umfasst, umfasst die folgenden Verfahrensschritte:

- Einteilen des Untersuchungsbereichs in mehrere Teilbereiche,
- Ermitteln von mehreren ersten Shimparametersätzen für den zumindest einen ersten Shimkanal, wobei jeweils ein erster Shimparametersatz der mehreren ersten Shimparametersätze für jeden der mehreren Teilbereiche ermittelt wird, wobei vor dem Ermitteln der mehreren ersten Shimparametersätze eine erste B0-Feldkarte erfasst wird, wobei das Ermitteln der mehreren ersten Shimparametersätze unter Verwendung der ersten B0-Feldkarte erfolgt,
- Ermitteln von einem zweiten Shimparametersatz für den zumindest einen zweiten Shimkanal unter Berücksichtigung der mehreren ersten ermittelten Shimparametersätze,
- Erfassen von Magnetresonanz-Bilddaten des Untersuchungsbereichs des Untersuchungsobjekts, wobei vor dem Erfassen der Magnetresonanz-Bilddaten der zumindest eine zweite Shimkanal anhand des zweiten Shimparametersatzes eingestellt wird und der zumindest eine erste Shimkanal für das Erfassen der Magnetresonanz-Bilddaten aus einem bestimmten Teilbereich der mehreren Teilbereiche anhand eines für den bestimmten Teilbereich ermittelten ersten Shimparametersatzes eingestellt wird. Das Verfahren ist dadurch gekennzeichnet, dass eine zweite BO-Feldkarte unter Verwendung der ersten BO-Feldkarte und der mehreren ersten Shimparametersätze berechnet wird, und die mehreren ersten Shimparametersätze bei dem Ermitteln des zweiten Shimparametersatzes derart berücksichtigt werden, dass der zweite Shimparametersatz unter Verwendung der zweiten BO-Feldkarte ermittelt wird.

Der Untersuchungsbereich, auch Aufnahmevolumen (field of view, FOV) genannt, stellt insbesondere ein Volumen dar, welches in den aufgenommenen Magnetresonanz-Bilddaten abgebildet wird. Der Untersuchungsbereich wird typischerweise durch einen Benutzer, beispielsweise auf einer Übersichtsaufnahme (Localizer) festgelegt. Selbstverständlich kann der Untersuchungsbereich alternativ oder zusätzlich auch automatisch, beispielsweise auf Grundlage eines ausgewählten Protokolls, festgelegt werden. Die mehreren Teilbereiche des Untersuchungsbereichs ergeben insbesondere zusammen den gesamten Untersuchungsbereich. Das Untersuchungsobjekt kann ein Patient, ein Proband, ein Tier oder ein Phantom sein. Die erfassten Magnetresonanz-Bilddaten werden insbesondere bereitgestellt, also einem Benutzer auf einer Anzeigeeinheit angezeigt und/oder in einer Datenbank abgespeichert.

Der Untersuchungsbereich weist gemäß dem vorgeschlagenen Vorgehen mehrere Teilbereiche, nämlich insbesondere mindestens einen ersten und einen zweiten Teilbereich, auf. Der Untersuchungsbereich kann selbstverständlich auch noch weitere Teilbereiche, insbesondere zusätzlich zum ersten und zweiten Teilbereich, aufweisen. Insbesondere kann eine einzelne Anregungsschicht der Magnetresonanz-Sequenz einen Teilbereich des Untersuchungsbereichs darstellen. Die Anzahl der Teilbereiche des Untersuchungsbereichs kann somit gleich der Anzahl der Schichten des Untersuchungsbereichs sein. Es sind selbstverständlich auch größere, beispielsweise mehrere Schichten umfassende, oder kleinere Teilbereiche des Untersuchungsbereichs denkbar. Das Erfassen der Magnetresonanz-Bilddaten aus den mehreren Teilbereichen des Untersuchungsbereichs erfolgt insbesondere separat für die mehreren Teilbereiche. Die vorliegende Erfindung ist insbesondere für solche Sequenztechniken relevant, bei denen Zeitintervalle in denen Magnetresonanz-Bilddaten aus dem ersten Teilbereich erfasst werden mit solchen Zeitintervallen aus denen Magnetresonanz-Bilddaten aus dem zweiten Teilbereich erfasst werden auf einer Zeitskala wechseln die klein gegenüber einer Einstellzeit des zumindest einen zweiten Shimkanals ist.

Die erste Shimkanalmenge kann einen ersten Shimkanal oder mehrere erste Shimkanäle umfassen. Ein erster Shimkanal der ersten Shimkanalmenge wird dabei insbesondere von einer Gradientenspule des Magnetresonanzgeräts gebildet. Es ist möglicherweise auch denkbar, dass ein erster Shimkanal der ersten Shimkanalmenge von einer dedizierten Shimspule der Shimeinheit gebildet wird. Der zumindest eine erste Shimkanal kann insbesondere Shimfelder erster Ordnung, insbesondere lineare Shimfelder, erzeugen. Theoretisch ist es auch denkbar, dass ein erster Shimkanal der ersten Shimkanalmenge Shimfelder höherer Ordnung, beispielsweise zweiter Ordnung, erzeugt.

Wie bereits beschrieben kann der zumindest eine erste Shimkanal während des Erfassens der Magnetresonanz-Bilddaten mittels unterschiedlicher Shimströme belegt werden. So können die Shimströme in dem zumindest einen ersten Shimkanal für ein Erfassen von Magnetresonanz-Bilddaten aus unterschiedlichen Teilbereichen des Untersuchungsbereichs umgeschaltet werden. So können für ein Erfassen der Magnetresonanz-Bilddaten aus einem ersten Teilbereich und einem zweiten Teilbereich des Untersuchungsbereichs verschiedene erste Shimparametersätze der mehreren ersten Shimparametersätze verwendet werden. Beispielsweise kann für ein Erfassen von Magnetresonanz-Bilddaten aus verschiedenen Schichten des Untersuchungsbereichs der zumindest eine erste Shimkanal mit unterschiedlichen Shimströmen belegt werden. Derart kann ein Feldbeitrag für eine Homogenisierung des Hauptmagnetfelds aufgrund der durch den zumindest einen ersten Shimkanal fließenden Shimströme während des Erfassens der Magnetresonanz-Bilddaten variiert werden. Der zumindest eine erste Shimkanal ist somit vorteilhafterweise zum dynamischen Shimmen ausgebildet.

Die zweite Shimkanalmenge kann einen zweiten Shimkanal oder mehrere zweite Shimkanäle umfassen. Ein solcher zweiter Shimkanal der zweiten Shimkanalmenge wird dabei insbesondere von einer dedizierten Shimspule gebildet. Der zumindest eine zweite Shimkanal kann insbesondere Shimfelder zweiter oder höherer Ordnung erzeugen. Theoretisch ist es auch denkbar, dass ein zweiter Shimkanal der zweiten Shimkanalmenge Shimfelder erster Ordnung erzeugt.

Wie bereits beschrieben wird der zumindest eine zweite Shimkanal während des Erfassens der Magnetresonanz-Bilddaten insbesondere stets mit den gleichen Shimströmen belegt. So kann für ein Erfassen von Magnetresonanz-Bilddaten aus verschiedenen Schichten des Untersuchungsbereichs der zumindest eine zweite Shimkanal mit den gleichen Shimströmen belegt werden. Derart kann insbesondere ein Feldbeitrag für eine Homogenisierung des Hauptmagnetfelds aufgrund der durch den zumindest einen zweiten Shimkanal fließenden Shimströme während des Erfassens der Magnetresonanz-Bilddaten konstant bleiben. Der zumindest eine zweite Shimkanal ist somit nicht zum dynamischen Shimmen ausgebildet.

Ein Shimparametersatz kann Einstellungen für eine Ansteuerung einer Shimeinheit des Magnetresonanzgeräts, insbesondere von zumindest einem bestimmten Shimkanal der Shimeinheit, umfassen. Beispielsweise kann ein Shimparametersatz einen Strom in dem zumindest einen Shimkanal festlegen. Eine Shim-Steuereinheit kann dann den zumindest einen Shimkanal bei der Aufnahme der Magnetresonanz-Bilddaten mit den durch den Shimparametersatz festgelegten Strom beaufschlagen. Des Weiteren kann ein Shimparametersatz einen geeigneten Wert für die Mittenfrequenz und/oder Resonanzfrequenz umfassen. Derart kann beispielsweise eine separate Frequenzjustage entfallen.

Das Ermitteln der mehreren ersten Shimparametersätze für den zumindest einen ersten Shimkanal erfolgt erfindungsgemäß unter Verwendung einer ersten B0-Feldkarte, wie in einem der folgenden Abschnitte noch beschrieben. Gemäß dem vorgeschlagenen Vorgehen gehen nun die mehreren ersten Shimparametersätze in das Ermitteln des zweiten Shimparametersatzes für den zumindest einen zweiten Shimkanal ein. So stellen die mehreren ersten Shimparametersätze Eingangsparameter für einen Algorithmus dar, welcher zum Ermitteln des zweiten Shimparametersatzes verwendet wird.

Die mehreren ersten Shimparametersätze sind erfindungsgemäß unterschiedlich zueinander ausgebildet. Sie weisen beispielsweise unterschiedliche Stromwerte für den zumindest einen Shimkanal auf. So umfassen die mehreren ersten Shimparametersätze einen ersten Teilbereich-Shimparametersatz und einen zweiten Teilbereich-Shimparametersatz. So wird der zumindest eine erste Shimkanal anhand der mehreren ersten Shimparametersätze derart eingestellt, dass für das Erfassen von Magnetresonanz-Bilddaten aus einem ersten Teilbereich der mehreren Teilbereiche der zumindest eine erste Shimkanal anhand des ersten Teilbereich-Shimparametersatz eingestellt wird und für das Erfassen von Magnetresonanz-Bilddaten aus einem zweiten Teilbereich der mehreren Teilbereiche der zumindest eine erste Shimkanal anhand des zweiten Teilbereich-Shimparametersatz eingestellt wird. Erfindungsgemäß wird für jeden Teilbereich der mehreren Teilbereiche ein erster Shimparametersatz der mehreren ersten Shimparametersätze ermittelt.

Dagegen wird insbesondere ein einzelner zweiter Shimparametersatz für den zumindest einen zweiten Shimkanal für das Erfassen der Magnetresonanz-Bilddaten ermittelt. Anhand dieses einzelnen zweiten Shimparametersatzes kann dann der zumindest eine zweite Shimkanal während des gesamten Erfassens der Magnetresonanz-Bilddaten aus den mehreren Teilbereichen des Untersuchungsbereichs, insbesondere aus dem ersten Teilbereich und dem zweiten Teilbereich, eingestellt werden. In speziellen Anwendungsfällen ist es möglicherweise auch denkbar, dass der zumindest eine zweite Shimkanal während des Erfassens der Magnetresonanz-Bilddaten mit unterschiedlichen Shimströmen belegt wird. Das kann bedeuten, dass mehrere zweite Shimparametersätze für den zumindest einen zweiten Shimkanal, insbesondere unter Berücksichtigung der mehreren ersten Shimparametersätze, ermittelt werden.

Das vorgeschlagene Vorgehen bietet den Vorteil, dass die Berücksichtigung der mehreren ersten Shimparametersätze bei dem Ermitteln des zweiten Shimparametersatzes zu einem besonders vorteilhaften zweiten Shimparametersatz führen kann. Der zweite Shimparametersatz kann so vorteilhafte, insbesondere optimale Shimströme, für den zumindest einen zweiten Shimkanal umfassen. Dabei kann der zumindest eine zweite Shimkanal insbesondere für das dynamische Shimmen nicht hinreichend schnell geschaltet werden. Mit der vorgeschlagenen Methode kann erreicht werden, dass Inhomogenitäten des Hauptmagnetfelds mittels eines Kompensationsgesamtfelds bestehend aus einem dynamisch geschalteten Kompensationsfeld des zumindest einen schnell schaltbaren ersten Shimkanals und einem konstanten Kompensationsfeld des zumindest einen zweiten Shimkanals höherer Ordnung besonders stark reduziert, insbesondere optimal kompensiert, werden können. Hierbei kann vorteilhafterweise im Mittel über alle Teilbereiche, insbesondere Schichten, des Untersuchungsbereichs eine gemessene Abweichung von einem optimalen konstanten Hauptmagnetfeld besonders stark kompensiert werden, insbesondere vollständiger als mit herkömmlichen Methoden. Derart kann eine hohe Bildqualität der so erfassten Magnetresonanz-Bilddaten aufgrund der besonders hohen Homogenität des Hauptmagnetfelds, insbesondere im Mittel über den gesamten Untersuchungsbereichs, erreicht werden. Beispielsweise können derart Artefakte in den Magnetresonanz-Bilddaten aufgrund von Inhomogenitäten des Hauptmagnetfelds vorteilhafterweise reduziert, insbesondere weitgehend vermieden werden.

In herkömmlichen dynamischen Shimverfahren erfolgt insbesondere das Ermitteln der mehreren ersten Shimparametersätze und des zweiten Shimparametersatzes unabhängig voneinander. Dagegen umfasst erfindungsgemäß, wie in einem der folgenden Abschnitte noch beschrieben, die Berücksichtigung der mehreren ersten Shimparametersätze bei dem Ermitteln des zweiten Shimparametersatzes eine Berücksichtigung von Feldbeiträgen der mehreren ersten Shimparametersätze.

Hierbei kann der Vorteil entstehen, dass ein bereits mittels der mehreren ersten Shimparametersätze korrigiertes virtuelles Hauptmagnetfeld eine höhere Homogenität und/oder eine geringere Variation aufweist als ein tatsächlich physikalisch vorliegendes Hauptmagnetfeld vor einem dynamischen Shimvorgang. Für ein solches mittels der mehreren ersten Shimparametersätze korrigiertes virtuelles Hauptmagnetfeld kann in der Regel einfacher und/oder besser ein zweiter Shimparametersatz für den zumindest einen statischen zweiten Shimkanal ermittelt werden, derart dass nach der Berücksichtigung von Feldbeiträgen des dynamischen Shimmens mittels des zumindest einen ersten Shimkanals verbleibende Inhomogenitäten des Hauptmagnetfelds über den gesamten Untersuchungsbereich vollständiger kompensiert werden können. Eine Variation des Hauptmagnetfelds innerhalb der einzelnen Teilbereiche des Untersuchungsbereichs kann dann vorteilhafterweise nach dem Einstellen des zumindest einen zweiten Shimkanals und der dynamischen Einstellung des zumindest einen ersten Shimkanals für die jeweilige Teilbereiche verringert werden.

Eine Ausführungsform sieht vor, dass die erste Shimkanalmenge und die zweite Shimkanalmenge disjunkt sind. Derart gehört insbesondere jeder Shimkanal der Shimeinheit entweder zur ersten Shimkanalmenge oder zur zweiten Shimkanalmenge. Die Shimparametersätze können so vorteilhafterweise separat für den zumindest einen ersten Shimkanal der ersten Shimkanalmenge und den zumindest einen zweiten Shimkanal der zweiten Shimkanalmenge berechnet werden.

Erfindungsgemäß wird vor dem Ermitteln der mehreren ersten Shimparametersätze eine erste B0-Feldkarte erfasst, wobei das Ermitteln der mehreren ersten Shimparametersätze unter Verwendung der ersten B0-Feldkarte erfolgt. Das Erfassen der ersten B0-Feldkarte kann eine Messung der ersten B0-Feldkarte, insbesondere mittels eines dem Fachmann geläufigen Verfahrens, umfassen. Eine BO-Feldkarte stellt eine Feldverteilung eines Hauptmagnetfelds des Magnetresonanzgeräts dar. Die BO-Feldkarte ist proportional zum Hauptmagnetfeld (B0-Feld) des Magnetresonanzgeräts. Diese BO-Feldkarte wird nun als erste BO-Feldkarte bezeichnet. Die BO-Feldkarte kann damit zum Identifizieren von Inhomogenitäten im Hauptmagnetfeld dienen, die insbesondere dann entstehen, wenn das Untersuchungsobjekt im Magnetresonanzgerät positioniert ist. Das Ermitteln der mehreren ersten Shimparametersätze kann dann derart anhand der ersten B0-Feldkarte erfolgen, dass die Inhomogenitäten des Hauptmagnetfelds mittels der auf den zumindest einen ersten Shimkanal angewendeten mehreren ersten Shimparametersätze während des Erfassens der Magnetresonanz-Bilddaten zumindest teilweise ausgeglichen werden. Dabei werden die Inhomogenitäten des Hauptmagnetfelds insbesondere spezifisch für den ersten Teilbereich und den zweiten Teilbereich des Untersuchungsbereichs ausgeglichen. Die erste BO-Feldkarte stellt derart einen vorteilhaften Ausgangspunkt zum Ermitteln der mehreren ersten Shimparametersätze dar.

Erfindungsgemäß wird eine zweite BO-Feldkarte unter Verwendung der ersten BO-Feldkarte und der mehreren ersten Shimparametersätze berechnet, wobei die mehreren ersten Shimparametersätze bei dem Ermitteln des zweiten Shimparametersatzes derart berücksichtigt werden, dass der zweite Shimparametersatz unter Verwendung der zweiten BO-Feldkarte ermittelt wird. Die mehreren ersten Shimparametersätze werden somit bei dem Ermitteln des zweiten Shimparametersatzes indirekt berücksichtigt, nämlich über die anhand der mehreren ersten Shimparametersätze berechneten zweiten B0-Feldkarte. Das Berechnen der zweiten BO-Feldkarte unter Verwendung der ersten BO-Feldkarte und der mehreren ersten Shimparametersätze kann beispielsweise wie im folgenden Abschnitt beschrieben erfolgen, allerdings auch mittels eines anderen, dem Fachmann als sinnvoll erscheinenden, Vorgehens. Über die berechnete zweite BO-Feldkarte können die mehreren ersten Shimparametersätze besonders vorteilhaft bei dem Ermitteln des zweiten Shimparametersatzes berücksichtigt werden.

Eine Ausführungsform sieht vor, dass die zweite BO-Feldkarte unter Verwendung der ersten BO-Feldkarte und der mehreren ersten Shimparametersätze derart berechnet wird, dass B0-Feldbeiträge, welche sich aus den mehreren ersten Shimparametersätzen ergeben, mit der ersten BO-Feldkarte verrechnet werden. Vorteilhafterweise werden dabei die BO-Feldbeiträge der jeweiligen Shimparametersätze separat für die Teilbereiche verrechnet. Dabei werden vorteilhafterweise ein erster räumlicher Teilbereich der zweiten BO-Feldkarte anhand des ersten räumlichen Teilbereiches der ersten BO-Feldkarte und des ersten Shimparametersatzes, welcher für den ersten Teilbereiches ermittelt wird, berechnet, wobei ein erster räumlicher Teilbereich der zweiten BO-Feldkarte erzeugt wird, und ein zweiter räumlicher Teilbereich der zweiten BO-Feldkarte anhand des zweiten Teilbereichs der ersten BO-Feldkarte und des ersten Shimparametersatzes, welcher für den zweiten räumlichen Teilbereiches bestimmt wird, berechnet, wobei ein zweiter räumlicher Teilbereich der zweiten BO-Feldkarte erzeugt wird. Derart kann der zweite Shimparametersatz unter Verwendung der zweiten BO-Feldkarte ermittelt werden. Die zweite B0-Feldkarte kann nach dem rechnerischen Entfernen der B0-Feldbeiträge der mehreren ersten Shimparametersätze aus der ersten BO-Feldkarte einen besonders vorteilhaften Ausgangspunkt zum Ermitteln des zweiten Shimparametersatzes darstellen. Derart kann nämlich der zweite Shimparametersatz die nach der Berücksichtigung der mehreren ersten Shimparametersätze verbleibenden Inhomogenitäten im Hauptmagnetfeld besonders vorteilhaft kompensieren.

Eine Ausführungsform sieht vor, dass ein räumlicher Abschnitt der ersten BO-Feldkarte mit den BO-Feldbeiträgen, welche sich aus einem geeigneten ersten Shimparametersatz der mehreren ersten Shimparametersätze ergeben, verrechnet wird, wobei der geeignete erste Shimparametersatz spezifisch für den Teilbereich der mehreren Teilbereiche, welcher zu dem räumlichen Abschnitt korrespondiert, ermittelt wurde. Vorteilhafterweise werden derart die BO-Feldbeiträge separat für verschiedene erste Shimparametersätze der mehreren ersten Shimparametersätze bei dem Ermitteln des zweiten Shimparametersatzes berücksichtigt.

Eine Ausführungsform sieht vor, eine dritte BO-Feldkarte unter Verwendung der ersten BO-Feldkarte und des zweiten Shimparametersatzes berechnet wird, wobei ein Ermitteln von mehreren angepassten ersten Shimparametersätzen für den zumindest einen ersten Shimkanal unter Verwendung der dritten B0-Feldkarte erfolgt, wobei für das Erfassen der Magnetresonanz-Bilddaten der zumindest eine erste Shimkanal anhand der mehreren angepassten ersten Shimparametersätze eingestellt wird. Die zweite BO-Feldkarte kann unter Verwendung des zweiten Shimparametersatzes derart angepasst werden, dass B0-Feldbeiträge, welche sich aus dem zweiten Shimparametersatz ergeben, mit der ersten BO-Feldkarte verrechnet werden. Derart werden nicht die ursprünglich auf Grundlage der ersten BO-Feldkarte ermittelten mehreren ersten Shimparametersätze, sondern die nochmals auf Grundlage der dritten BO-Feldkarte ermittelten angepassten mehreren ersten Shimparametersätze bei dem Erfassen der Magnetresonanz-Bilddaten für den zumindest einen ersten Shimkanal gesetzt. Die mehreren angepassten ersten Shimparametersätze können dabei die für die mehreren Teilbereiche des Untersuchungsbereichs vorliegenden Inhomogenitäten des Hauptmagnetfelds insbesondere noch besser kompensieren als die mehreren ersten Shimparametersätze. Ein Grund dafür ist, dass die durch den zumindest einen zweiten Shimkanal induzierten Kompensationsfelder derart spezifisch für die mehreren Teilbereiche des Untersuchungsbereichs und nicht nur global berücksichtigt werden können. Dafür ist keine zusätzliche Messzeit nötig, da die dritte BO-Feldkarte aus der bereits gemessenen ersten BO-Feldkarte berechnet werden kann.

Eine Ausführungsform sieht vor, dass für das Erfassen der Magnetresonanz-Bilddaten zunächst der zumindest eine zweite Shimkanal anhand des zweiten Shimparametersatzes eingestellt wird, anschließend der zumindest eine erste Shimkanal anhand eines ersten Shimparametersatzes, welcher für einen ersten Teilbereich der mehreren Teilbereiche ermittelt wird, eingestellt wird, anschließend Magnetresonanz-Bilddaten aus dem ersten Teilbereich erfasst werden, anschließend der zumindest eine erste Shimkanal anhand eines ersten Shimparametersatzes, welcher für einen zweiten Teilbereich der mehreren Teilbereiche ermittelt wird, eingestellt wird, anschließend Magnetresonanz-Bilddaten aus dem zweiten Teilbereich erfasst werden. Der zumindest eine zweite Shimkanal wird derart insbesondere für das Erfassen der Magnetresonanz-Bilddaten aus dem ersten Teilbereich und dem zweiten Teilbereich anhand des zweiten Shimparametersatzes eingestellt. Der zumindest eine erste Shimkanal wird insbesondere für das Erfassen der Magnetresonanz-Bilddaten anhand des ersten Shimparametersatzes, der für den ersten Teilbereich ermittelt wurde, eingestellt. Für das Erfassen von Magnetresonanz-Bilddaten aus dem zweiten Teilbereich wird insbesondere der zumindest eine erste Shimkanal anhand des für den zweiten Teilbereich ermittelten ersten Shimparametersatzes eingestellt. Derart können für die verschiedenen Teilbereiche des Untersuchungsbereiches Inhomogenitäten des Hauptmagnetfelds besonders vorteilhaft kompensiert werden.

Eine Ausführungsform sieht vor, dass zwischen dem Einstellen des zumindest einen zweiten Shimkanals und dem Erfassen der Magnetresonanz-Bilddaten aus dem ersten Teilbereich eine zweite Einschwingzeit vergeht und zwischen dem Einstellen des zumindest einen ersten Shimkanals anhand des ersten Shimparametersatzes und dem Erfassen der Magnetresonanz-Bilddaten aus dem ersten Teilbereich eine erste Einschwingzeit vergeht, wobei die erste Einschwingzeit kürzer als die zweite Einschwingzeit ist. Die Einschwingzeit (settling time) ist dabei insbesondere diejenige Zeit, welche zwischen einem Einstellen eines Shimkanals mit einem Strom und einer Einstellung eines gewünschten Feldbeitrags des Shimkanals vergeht. Der zumindest eine erste Shimkanal kann derart schneller als der zumindest eine zweite Shimkanal eingestellt werden. Somit kann der zumindest eine erste Shimkanal insbesondere dynamisch eingestellt werden.

Eine Ausführungsform sieht vor, dass nach dem Einstellen des zumindest einen zweiten Shimkanals eine vierte BO-Feldkarte erfasst wird, wobei ein Ermitteln von mehreren veränderten ersten Shimparametersätzen für den zumindest einen ersten Shimkanal unter Verwendung der vierten BO-Feldkarte erfolgt, wobei während des Erfassens der Magnetresonanz-Bilddaten der zumindest eine erste Shimkanal anhand der mehreren veränderten ersten Shimparametersätze eingestellt wird. Die zusätzliche Messzeit zum Erfassen der vierten BO-Feldkarte kann sich lohnen, wenn die durch den zumindest einen zweiten Shimkanal tatsächlich induzierten Kompensationsfelder von theoretischen und/oder berechneten Kompensationsfeldern (siehe die Berechnung der dritten BO-Feldkarte im vor-vorletzten Abschnitt) abweichen.

Eine Ausführungsform sieht vor, dass das Erfassen der ersten BO-Feldkarte unter Verwendung von Rohdaten erfolgt, welche aus mindestens drei Echosignalen bestehen, die jeweils nach einer Anregung des Untersuchungsbereichs akquiriert werden. Auf diese Weise kann auch die erwähnte vierte BO-Feldkarte erfasst werden. Durch eine spezielle Wahl der Echozeitdifferenz zwischen ersten und letzten Echosignal kann erreicht werden das B0 Feldkarten die aus Phasen-Differenzbilder des ersten und letzten Echosignals berechnet werden nur von der lokalen B0-Abweichung abhängen und nicht von der spektralen Zusammensetzung des Gewebes. Die aus der Phasendifferenz des ersten und letzten Echosignal berechnete B0-Karte kann anschließend entpackt werden (engl. "unwrapped"), d.h. Phasenumschläge in Folge der 2n Periodizität der Phase können rechnerisch entfernt werden. Die Signale der Zwischenechos können unter anderem zu einer absoluten Kalibrierung der entpackten BO-Feldkarte eingesetzt werden. Dadurch gelingt es eine BO-Feldkarte zu berechnen in der ein Pixel-Wert proportional ist zur lokalen absoluten Abweichung von der Resonanzfrequenz. Dies hat den Vorteil dass auch die RF-Mittenfrequenz (Shimkanal 0-ter Ordnung) direkt aus der B0-Feldkarte bestimmt werden kann. Auf eine separate Frequenzjustage innerhalb der jeweiligen Teilbereiche kann damit verzichtet werden.

Das erfindungsgemäße Magnetresonanzgerät umfasst eine Shimeinheit, welche eine erste Shimkanalmenge mit zumindest einem ersten Shimkanal und eine zweite Shimkanalmenge mit zumindest einem zweiten Shimkanal umfasst, eine Bilddatenerfassungseinheit und eine Recheneinheit mit einer Einteilungseinheit, einer ersten Ermittlungseinheit und einer zweiten Ermittlungseinheit, wobei das Magnetresonanzgerät dazu ausgebildet ist, ein erfindungsgemäßes Verfahren auszuführen.

Derart ist das Magnetresonanzgerät zum Ausführen eines Verfahrens zur Magnetresonanz-Bildgebung in einem Untersuchungsbereich eines Untersuchungsobjekts ausgebildet. Die Einteilungseinheit ist zum Einteilen des Untersuchungsbereichs in mehrere Teilbereiche ausgebildet. Die erste Ermittlungseinheit ist zu einem Ermitteln von mehreren ersten Shimparametersätzen für den zumindest einen ersten Shimkanal, wobei jeweils ein erster Shimparametersatz der mehreren ersten Shimparametersätze für jeden der mehreren Teilbereiche ermittelt wird, ausgebildet. Die zweite Ermittlungseinheit ist zu einem Ermitteln von einem zweiten Shimparametersatz für den zumindest einen zweiten Shimkanal unter Berücksichtigung der mehreren ersten ermittelten Shimparametersätze ausgebildet. Die Bilddatenerfassungseinheit ist zu einem Erfassen von Magnetresonanz-Bilddaten des Untersuchungsbereichs des Untersuchungsobjekts, wobei vor dem Erfassen der Magnetresonanz-Bilddaten der zumindest eine zweite Shimkanal anhand des zweiten Shimparametersatzes eingestellt wird und der zumindest eine erste Shimkanal für das Erfassen der Magnetresonanz-Bilddaten aus einem bestimmten Teilbereich der mehreren Teilbereiche anhand eines für den bestimmten Teilbereich ermittelten ersten Shimparametersatzes eingestellt wird.

Gemäß einer Ausführungsform des Magnetresonanzgeräts ist die Shimeinheit derart ausgebildet, dass die erste Shimkanalmenge und die zweite Shimkanalmenge disjunkt sind.

Erfindungsgemäß umfasst das Magnetresonanzgerät eine Feldkartenerfassungseinheit, wobei die Feldkartenerfassungseinheit und die erste Ermittlungseinheit derart ausgebildet sind, dass vor dem Ermitteln der mehreren ersten Shimparametersätze eine erste B0-Feldkarte erfasst wird, wobei das Ermitteln der mehreren ersten Shimparametersätze unter Verwendung der ersten B0-Feldkarte erfolgt.

Erfindungsgemäß umfasst das Magnetresonanzgerät eine Feldkartenerfassungseinheit, wobei die Feldkartenerfassungseinheit, die erste Ermittlungseinheit und die zweite Ermittlungseinheit derart ausgebildet sind, dass eine zweite BO-Feldkarte unter Verwendung der ersten BO-Feldkarte und der mehreren ersten Shimparametersätze berechnet wird, und wobei die mehreren ersten Shimparametersätze bei dem Ermitteln des zweiten Shimparametersatzes derart berücksichtigt werden, dass der zweite Shimparametersatz unter Verwendung der zweiten BO-Feldkarte ermittelt wird.

Gemäß einer Ausführungsform des Magnetresonanzgeräts umfasst das Magnetresonanzgerät eine Feldkartenerfassungseinheit, wobei die Feldkartenerfassungseinheit derart ausgebildet ist, dass die zweite BO-Feldkarte unter Verwendung der ersten B0-Feldkarte und der mehreren ersten Shimparametersätze derart berechnet wird, dass B0-Feldbeiträge, welche sich aus den mehreren ersten Shimparametersätzen ergeben, mit der ersten BO-Feldkarte verrechnet werden.

Gemäß einer Ausführungsform des Magnetresonanzgeräts umfasst das Magnetresonanzgerät eine Feldkartenerfassungseinheit, wobei die Feldkartenerfassungseinheit derart ausgebildet ist, dass ein räumlicher Abschnitt der ersten BO-Feldkarte mit den BO-Feldbeiträgen, welche sich aus einem geeigneten ersten Shimparametersatz der mehreren ersten Shimparametersätze ergeben, verrechnet wird, wobei der geeignete erste Shimparametersatz spezifisch für den Teilbereich der mehreren Teilbereiche, welcher zu dem räumlichen Abschnitt korrespondiert, ermittelt wurde.

Gemäß einer Ausführungsform des Magnetresonanzgeräts umfasst das Magnetresonanzgerät eine Feldkartenerfassungseinheit, wobei die Bilddatenerfassungseinheit, die erste Ermittlungseinheit und die Feldkartenerfassungseinheit derart ausgebildet sind, dass eine dritte BO-Feldkarte unter Verwendung der ersten BO-Feldkarte und des zweiten Shimparametersatzes berechnet wird, wobei ein Ermitteln von mehreren angepassten ersten Shimparametersätzen für den zumindest einen ersten Shimkanal unter Verwendung der dritten BO-Feldkarte erfolgt, wobei für das Erfassen der Magnetresonanz-Bilddaten der zumindest eine erste Shimkanal anhand der mehreren angepassten ersten Shimparametersätze eingestellt wird.

Gemäß einer Ausführungsform des Magnetresonanzgeräts ist die Bilddatenerfassungseinheit derart ausgebildet, dass für das Erfassen der Magnetresonanz-Bilddaten zunächst der zumindest eine zweite Shimkanal anhand des zweiten Shimparametersatzes eingestellt wird, anschließend der zumindest eine erste Shimkanal anhand eines ersten Shimparametersatzes, welcher für einen ersten Teilbereich der mehreren Teilbereiche ermittelt wird, eingestellt wird, anschließend Magnetresonanz-Bilddaten aus dem ersten Teilbereich erfasst werden, anschließend der zumindest eine erste Shimkanal anhand eines ersten Shimparametersatzes, welcher für einen zweiten Teilbereich der mehreren Teilbereiche ermittelt wird, eingestellt wird, anschließend Magnetresonanz-Bilddaten aus dem zweiten Teilbereich erfasst werden.

Gemäß einer Ausführungsform des Magnetresonanzgeräts ist die Bilddatenerfassungseinheit derart ausgebildet, dass zwischen dem Einstellen des zumindest einen zweiten Shimkanals und dem Erfassen der Magnetresonanz-Bilddaten aus dem ersten Teilbereich eine zweite Einschwingzeit vergeht und zwischen dem Einstellen des zumindest einen ersten Shimkanals anhand des ersten Shimparametersatzes und dem Erfassen der Magnetresonanz-Bilddaten aus dem ersten Teilbereich eine erste Einschwingzeit vergeht, wobei die erste Einschwingzeit kürzer als die zweite Einschwingzeit ist.

Gemäß einer Ausführungsform des Magnetresonanzgeräts umfasst das Magnetresonanzgerät eine Feldkartenerfassungseinheit, wobei die Feldkartenerfassungseinheit und die Bilddatenerfassungseinheit derart ausgebildet sind, dass nach dem Einstellen des zumindest einen zweiten Shimkanals eine vierte B0-Feldkarte erfasst wird, wobei ein Ermitteln von mehreren veränderten ersten Shimparametersätzen für den zumindest einen ersten Shimkanal unter Verwendung der vierten BO-Feldkarte erfolgt, wobei während des Erfassens der Magnetresonanz-Bilddaten der zumindest eine erste Shimkanal anhand der mehreren veränderten ersten Shimparametersätze eingestellt wird.

Gemäß einer Ausführungsform des Magnetresonanzgeräts umfasst das Magnetresonanzgerät eine Feldkartenerfassungseinheit, wobei die Feldkartenerfassungseinheit derart ausgebildet ist, dass das Erfassen der ersten BO-Feldkarte unter Verwendung von Rohdaten erfolgt, welche aus mindestens drei Echosignalen bestehen, die jeweils nach einer Anregung des Untersuchungsbereichs akquiriert werden.

Das erfindungsgemäße Computerprogrammprodukt ist direkt in einen Speicher einer programmierbaren Recheneinheit eines Magnetresonanzgeräts ladbar und weist Programmcode-Mittel auf, um ein erfindungsgemäßes Verfahren auszuführen, wenn das Computerprogrammprodukt in der Recheneinheit des erfindungsgemäßen Magnetresonanzgeräts ausgeführt wird. Dadurch kann das erfindungsgemäße Verfahren schnell, identisch wiederholbar und robust ausgeführt werden. Das Computerprogrammprodukt ist so konfiguriert, dass es mittels der Recheneinheit die erfindungsgemäßen Verfahrensschritte ausführen kann. Die Recheneinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem computerlesbaren Medium gespeichert oder auf einem Netzwerk oder Server hinterlegt, von wo es in den Prozessor einer lokalen Recheneinheit geladen werden kann, der mit dem Magnetresonanzgeräts direkt verbunden oder als Teil des Magnetresonanzgeräts ausgebildet sein kann. Weiterhin können Steuerinformationen des Computerprogrammprodukts auf einem elektronisch lesbaren Datenträger gespeichert sein. Die Steuerinformationen des elektronisch lesbaren Datenträgers sind derart ausgestaltet, dass sie bei Verwendung des Datenträgers in einer Recheneinheit des erfindungsgemäßen Magnetresonanzgeräts ein erfindungsgemäßes Verfahren durchführen. Beispiele für elektronische lesbare Datenträger sind eine DVD, ein Magnetband oder einen USB-Stick, auf welchem elektronisch lesbare Steuerinformationen, insbesondere Software (vgl. oben), gespeichert ist. Wenn diese Steuerinformationen (Software) von dem Datenträger gelesen und in eine Steuerung und/oder Recheneinheit des Magnetresonanzgeräts gespeichert werden, können alle erfindungsgemäßen Ausführungsformen der vorab beschriebenen Verfahren durchgeführt werden. So kann die Erfindung auch von dem besagten computerlesbaren Medium und/oder dem besagten elektronisch lesbaren Datenträger ausgehen.

Die Vorteile des erfindungsgemäßen Magnetresonanzgeräts und des erfindungsgemäßen Computerprogrammprodukts entsprechen im Wesentlichen den Vorteilen des erfindungsgemäßen Verfahrens, welche vorab im Detail ausgeführt sind. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module, insbesondere durch Hardware-Module, ausgebildet.

Im Folgenden wird die Erfindung anhand der in den Figuren dargestellten Ausführungsbeispiele näher beschrieben und erläutert.

Es zeigen:

Fig. 1    ein erfindungsgemäßes Magnetresonanzgerät in einer schematischen Darstellung,

Fig. 2    ein Ablaufdiagramm eines Verfahrens, das einen Teil der Verfahrensschritte eines erfindungsgemäßen Verfahrens enthält, wobei das in der Fig. 2 gezeigte Verfahren als solches nicht Teil der Erfindung ist,

Fig. 3    ein Ablaufdiagramm einer ersten Ausführungsform eines erfindungsgemäßen Verfahrens,

Fig. 4    ein Ablaufdiagramm einer zweiten Ausführungsform eines erfindungsgemäßen Verfahrens und

Fig. 5    ein Ablaufdiagramm einer dritten Ausführungsform eines erfindungsgemäßen Verfahrens.

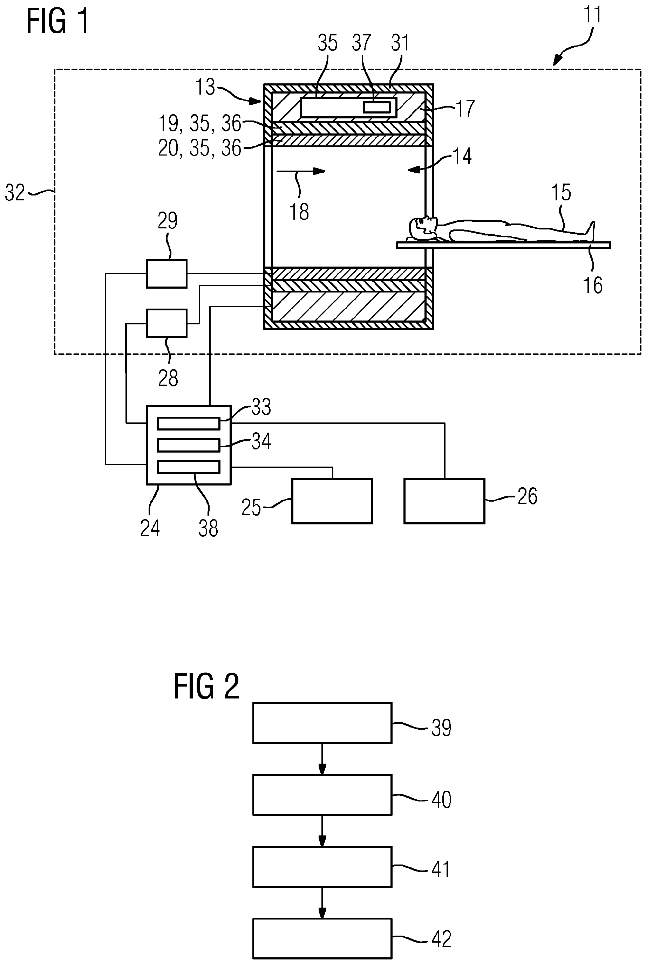

**Fig. 1** stellt ein erfindungsgemäßes Magnetresonanzgerät 11 schematisch dar. Das Magnetresonanzgerät 11 umfasst eine von einer Magneteinheit 13 gebildete Detektoreinheit mit einem Hauptmagneten 17 zu einem Erzeugen eines starken und insbesondere konstanten Hauptmagnetfelds 18. Zudem weist das Magnetresonanzgerät 11 einen zylinderförmigen Patientenaufnahmebereich 14 zu einer Aufnahme eines Untersuchungsobjekts 15, im vorliegenden Fall eines Patienten, auf, wobei der Patientenaufnahmebereich 14 in einer Umfangsrichtung von der Magneteinheit 13 zylinderförmig umschlossen ist. Der Patient 15 kann mittels einer Patientenlagerungsvorrichtung 16 des Magnetresonanzgeräts 11 in den Patientenaufnahmebereich 14 geschoben werden. Die Patientenlagerungsvorrichtung 16 weist hierzu einen Liegentisch auf, der bewegbar innerhalb des Magnetresonanzgeräts 11 angeordnet ist. Die Magneteinheit 13 ist mittels einer Gehäuseverkleidung 31 des Magnetresonanzgeräts nach außen abgeschirmt.

Die Magneteinheit 13 weist weiterhin eine Gradientenspuleneinheit 19 zu einer Erzeugung von Magnetfeldgradienten auf, die für eine Ortskodierung während einer Bildgebung verwendet werden. Die Gradientenspuleneinheit 19 wird mittels einer Gradientensteuereinheit 28 angesteuert. Des Weiteren weist die Magneteinheit 13 eine Hochfrequenzantenneneinheit 20, welche im gezeigten Fall als fest in das Magnetresonanzgerät 10 integrierte Körperspule ausgebildet ist, und eine Hochfrequenzantennensteuereinheit 29 zu einer Anregung einer Polarisation, die sich in dem von dem Hauptmagneten 17 erzeugten Hauptmagnetfeld 18 einstellt, auf. Die Hochfrequenzantenneneinheit 20 wird von der Hochfrequenzantennensteuereinheit 29 angesteuert und strahlt hochfrequente Magnetresonanz-Sequenzen in einen Untersuchungsraum, der im Wesentlichen von dem Patientenaufnahmebereich 14 gebildet ist, ein. Die Hochfrequenzantenneneinheit 20 ist weiterhin zum Empfang von Magnetresonanz-Signalen, insbesondere aus dem Patienten 15, ausgebildet.

Zu einer Steuerung des Hauptmagneten 17, der Gradientensteuereinheit 28 und der Hochfrequenzantennensteuereinheit 29 weist das Magnetresonanzgerät 11 eine Recheneinheit 24 auf. Die Recheneinheit 24 steuert zentral das Magnetresonanzgerät 11, wie beispielsweise das Durchführen einer vorbestimmten bildgebenden Gradientenechosequenz. Steuerinformationen wie beispielsweise Bildgebungsparameter, sowie rekonstruierte Magnetresonanz-Bilder können auf einer Bereitstellungseinheit 25, im vorliegenden Fall einer Anzeigeeinheit 25, des Magnetresonanzgeräts 11 für einen Benutzer bereitgestellt werden. Zudem weist das Magnetresonanzgerät 11 eine Eingabeeinheit 26 auf, mittels derer Informationen und/oder Parameter während eines Messvorgangs von einem Benutzer eingegeben werden können. Die Recheneinheit 24 kann die Gradientensteuereinheit 28 und/oder Hochfrequenzantennensteuereinheit 29 und/oder die Anzeigeeinheit 25 und/oder die Eingabeeinheit 26 umfassen. Die Recheneinheit 24 umfasst im dargestellten Fall eine Einteilungseinheit 38, eine erste Ermittlungseinheit 33 und eine zweite Ermittlungseinheit 34.

Das Magnetresonanzgerät umfasst weiterhin eine Shimeinheit 35, welche eine erste Shimkanalmenge mit zumindest einem ersten Shimkanal 36 und eine zweite Shimkanalmenge mit zumindest einem zweiten Shimkanal 37 umfasst.

Die erste Shimkanalmenge umfasst beispielsweise drei erste Shimkanäle 36. Die drei ersten Shimkanäle 36 werden im in Fig. 1 gezeigten Fall von den drei Gradientenspulen der Gradientenspuleneinheit 19 gebildet. Diese drei ersten Shimkanäle 36 können Shimfelder in x-Richtung, y-Richtung und in z-Richtung erzeugen. Dabei verläuft exemplarisch die x-Richtung entlang einer horizontalen Körperachse eines auf dem Rücken liegenden Untersuchungsobjekts 15, die y-Richtung entlang der vertikalen Körperachse des Untersuchungsobjekts 15 und die z-Richtung entlang einer Sagittalachse des Untersuchungsobjekts 15. Die Gradientenspulen und der Gradientenverstärker sind aus anderen Gründen so ausgelegt, dass die Gradientenfelder auf einer Zeitskala von wenigen Mikrosekunden variiert werden können. Diese Zeit ist also klein gegenüber der Zeit die zwischen Akquisition von Daten aus verschiedenen Teilbereichen und/oder Schichten eines Untersuchungsbereichs vergeht. Die drei ersten Shimkanäle 36 erster Ordnung kann man also typischerweise für dynamisches Shimmen verwenden. Des Weiteren umfasst die erste Shimkanal-menge eine RF-Mittenfrequenz. Diese RF-Mittenfrequenz gibt die mittlere Abweichung der Resonanzfrequenz innerhalb des Teilbereiches, dem der jeweilige erste Shimparametersatz zugeordnet ist, von der RF-Mittenfrequenz, die bei der Akquisition der ersten B0-Feldkarte eingestellt war, wieder. Die RF-Mittenfrequenz kann formal wie ein Shimkanal, nämlich ein Shimkanal 0-ter Ordnung, behandelt werden.

Weiterhin umfasst die zweite Shimkanalmenge beispielsweise fünf dedizierte zweite Shimkanäle 37. Diese fünf zweiten Shimkanäle 37 können Shimfelder zweiter Ordnung erzeugen. Diese Shimfelder sind insbesondere proportional zu dem durch die zweiten Shimkanäle 37 fließenden Strom und der räumliche Verlauf der Shimfelder kann beispielsweise in guter Näherung durch $z^2 - (x^2+y^2)/2)$, xz, yz, $(x^2-y^2)/2$ und xy beschrieben werden. Die zu zweiten Shimkanäle 37 zugehörigen Verstärker sind typischerweise derart ausgelegt das zwischen dem Ändern des Stroms durch den zweiten Shimkanal 37 und dem Einstellen des gewünschten Feldes eine Einschwingzeit (settling time) in einer Größenordnung einer Sekunde oder länger vergeht. Diese Einschwingzeit ist damit länger als die typische Zeit zwischen der Akquisition von Daten aus verschiedenen Teilbereichen und/oder Schichten eines Untersuchungsbereichs. Die fünf zweiten Shimkanäle 37 zweiter Ordnung kann man also typischerweise nicht für dynamisches Shimmen verwenden. Selbstverständlich können die zweiten Shimkanäle 37 auch Shimkanäle einer höheren Ordnung enthalten, die beispielsweise Shimfelder einer dritten oder vierten Ordnung erzeugen. Die erste Shimkanalmenge und die zweite Shimkanalmenge sind im gezeigten Fall disjunkt ausgebildet.

Das Magnetresonanzgerät 11 umfasst weiterhin eine Bilddatenerfassungseinheit 32. Die Bilddatenerfassungseinheit 32 wird im vorliegenden Fall von der Magneteinheit 13 zusammen mit der Hochfrequenzantennensteuereinheit 29 und der Gradientensteuereinheit 28 gebildet. Das Magnetresonanzgerät 11 ist somit zusammen mit der Bilddatenerfassungseinheit 32, der Recheneinheit 24 und der Shimeinheit 35 zur Ausführung eines erfindungsgemäßen Verfahrens zur Magnetresonanz-Bildgebung ausgelegt.

Das dargestellte Magnetresonanzgerät 11 kann selbstverständlich weitere Komponenten umfassen, die Magnetresonanzgeräte 11 gewöhnlich aufweisen. Eine allgemeine Funktionsweise eines Magnetresonanzgeräts 11 ist zudem dem Fachmann bekannt, so dass auf eine detaillierte Beschreibung der weiteren Komponenten verzichtet wird.

**Fig. 2** zeigt ein Ablaufdiagramm eines Verfahrens zur Magnetresonanz-Bildgebung in einem Untersuchungsbereich eines Untersuchungsobjekts 15 mittels eines Magnetresonanzgeräts 11. Das gezeigte Verfahren beinhaltet einen Teil der Verfahrensschritte eines erfindungsgemäßen Verfahrens und ist als solches nicht Teil der Erfindung.

In einem ersten Verfahrensschritt 39 erfolgt ein Einteilen des Untersuchungsbereichs in mehrere Teilbereiche mittels der Einteilungseinheit 38. Beispielsweise bildet jede Anregungsschicht eines Schichtstapels einen Teilbereich der mehreren Teilbereiche.

In einem weiteren Verfahrensschritt 40 erfolgt ein Ermitteln von mehreren ersten Shimparametersätzen für den zumindest einen ersten Shimkanal 36, wobei jeweils ein erster Shimparametersatz der mehreren ersten Shimparametersätze für die mehreren Teilbereiche ermittelt wird, mittels der ersten Ermittlungseinheit 33.

In einem weiteren Verfahrensschritt 41 erfolgt ein Ermitteln von mehreren zweiten Shimparametersätzen für den zumindest einen zweiten Shimkanal 37 unter Berücksichtigung der mehreren ersten ermittelten Shimparametersätze mittels der zweiten Ermittlungseinheit 34.

In einem weiteren Verfahrensschritt 42 erfolgt ein Erfassen von Magnetresonanz-Bilddaten des Untersuchungsbereichs des Untersuchungsobjekts 15 mittels der Bilddatenerfassungseinheit 32, wobei vor dem Erfassen der Magnetresonanz-Bilddaten der zumindest eine zweite Shimkanal 37 anhand des zweiten Shimparametersatzes eingestellt wird und der zumindest eine erste Shimkanal 36 für das Erfassen der Magnetresonanz-Bilddaten aus einem bestimmten Teilbereich der mehreren Teilbereiche anhand eines für den bestimmten Teilbereich ermittelten ersten Shimparametersatzes eingestellt wird.

Die erfassten Magnetresonanz-Bilddaten können auf der Anzeigeeinheit 25 ausgegeben werden und/oder in einer Datenbank abgespeichert werden.

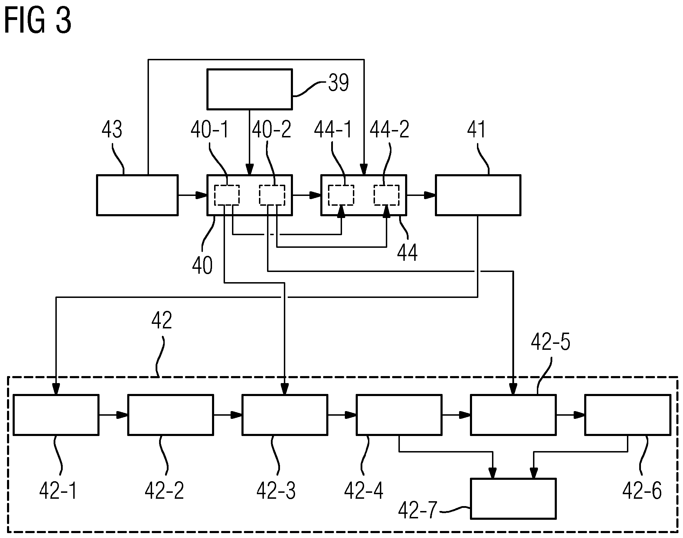

**Fig. 3** zeigt ein Ablaufdiagramm einer ersten Ausführungsform eines erfindungsgemäßen Verfahrens zur Magnetresonanz-Bildgebung in einem Untersuchungsbereich eines Untersuchungsobjekts 15 mittels eines Magnetresonanzgeräts 11.

Die nachfolgende Beschreibung beschränkt sich im Wesentlichen auf die Unterschiede zu dem Beispiel in Fig. 2, wobei bezüglich gleich bleibender Verfahrensschritte auf die Beschreibung des Beispiels in Fig. 2 verwiesen wird. Im Wesentlichen gleich bleibende Verfahrensschritte sind grundsätzlich mit den gleichen Bezugszeichen beziffert.

Die in Fig. 3 gezeigte Ausführungsform des erfindungsgemäßen Verfahrens umfasst die Verfahrensschritte 39, 40, 41, 42 der ersten Ausführungsform des Verfahrens gemäß Fig. 2. Zusätzlich umfasst die in Fig. 3 gezeigte Ausführungsform des erfindungsgemäßen Verfahrens zusätzliche Verfahrensschritte und Unterschritte. Denkbar ist auch ein zu Fig. 3 alternativer Verfahrensablauf, welcher nur einen Teil der in Fig. 2 dargestellten zusätzlichen Verfahrensschritte und/oder Unterschritte aufweist, sofern der alternative Verfahrensablauf in den Schutzumfang der Erfindung fällt, wie er von den Ansprüchen definiert wird. Selbstverständlich kann auch ein zu Fig. 3 alternativer Verfahrensablauf zusätzliche Verfahrensschritte und/oder Unterschritte aufweisen.

Im Beispiel der Fig.3 wird für eine bessere Übersicht angenommen, dass im ersten Verfahrensschritt 39 der Untersuchungsbereich in zwei Teilbereiche eingeteilt wird, also beispielsweise aus zwei Einzelschichten besteht. Das Konzept lässt sich natürlich auf eine Einteilung in mehr Teilbereiche beliebig erweitern.

In einem weiteren Verfahrensschritt 43 wird vor dem Ermitteln der mehreren ersten Shimparametersätze eine erste B0-Feldkarte erfasst. Die erste BO-Feldkarte gibt insbesondere lokale Abweichungen von einem idealen konstanten Hauptmagnetfeld 18 innerhalb der mehreren Teilbereiche des Untersuchungsbereichs wider. Die BO-Feldkarte kann auch eine Frequenzkarte umfassen, welche eine lokale Abweichung einer Resonanzfrequenz von einer Systemfrequenz widergibt. Eine Frequenzkarte $\Delta f(x,y,z)$ und eine BO-Feldkarte $AB_0(x,y,z)$ lassen sich typischerweise derart umrechnen:

$$\Delta f(x,y,z) = (\gamma/(2\pi)) \times \Delta B_0(x,y,z)$$

Dabei ist $\gamma/(2\pi)$ das gyromagnetische Verhältnis (gyromagnetic ratio), welches für Protonen 42.576 MHz/T beträgt.

Das Ermitteln der mehreren ersten Shimparametersätze im weiteren Verfahrensschritt 40 erfolgt unter Verwendung der ersten BO-Feldkarte. Es werden hierbei in einem ersten Teilschritt 40-1 des weiteren Verfahrensschritts 40 ein erster Teilbereich-Shimparametersatz anhand der ersten BO-Feldkarte ermittelt und in einem zweiten Teilschritt 40-2 des weiteren Verfahrensschritts 40 ein zweiter Teilbereich-Shimparametersatz anhand der ersten BO-Feldkarte ermittelt. Der erste Teilbereich-Shimparametersatz ist ein erster Shimparametersatz, der für den ersten Teilbereich ermittelt wurde. Der zweite Teilbereich-Shimparametersatz ist ein erster Shimparametersatz, der für den zweiten Teilbereich ermittelt wurde. Selbstverständlich können auch noch weitere Teilbereich-Shimdatensätze ermittelt werden. Insbesondere kann für jede Schicht des Untersuchungsbereichs ein Teilbereich-Shimparametersatz ermittelt werden.

Der erste Shimparametersatz, der für den ersten Teilbereich ermittelt wurde, $\Delta f_0^{(1)}, \Delta G_x^{(1)}, \Delta G_y^{(1)}, \Delta G_z^{(1)}$ umfasst insbesondere eine erste Resonanzfrequenz $\Delta f_0^{(1)}$ für die Frequenzjustage, einen ersten x-Gradientenoffsetstrom $\Delta G_x^{(1)}$ für die Gradientenspule in x-Richtung, einen ersten y-Gradientenoffsetstrom $\Delta G_y^{(1)}$ für die Gradientenspule in y-Richtung und einen ersten z-Gradientenoffsetstrom $\Delta G_z^{(1)}$ für die Gradientenspule in z-Richtung. Der erste Shimparametersatz, der für den ersten Teilbereich ermittelt wurde, $\Delta f_0^{(1)}, \Delta G_x^{(1)}, \Delta G_y^{(1)}, \Delta G_z^{(1)}$ wird dabei vorteilhafterweise derart gewählt, dass lokale Feldabweichungen $AB_0(x,y,z)$ im ersten Teilbereich optimal kompensiert werden.

Der erste Shimparametersatz, der für den zweiten Teilbereich ermittelt wurde, $\Delta f_0^{(2)}, \Delta G_x^{(2)}, \Delta G_y^{(2)}, \Delta G_z^{(2)}$ umfasst insbesondere eine zweite Resonanzfrequenz $\Delta f_0^{(2)}$ für die Frequenzjustage, einen zweiten x-Gradientenoffsetstrom $\Delta G_x^{(2)}$ für die Gradientenspule in x-Richtung, einen zweiten y-Gradientenoffsetstrom $\Delta G_y^{(2)}$ für die Gradientenspule in y-Richtung und einen zweiten z-Gradientenoffsetstrom $\Delta G_z^{(2)}$ für die Gradientenspule in z-Richtung. Der erste Shimparametersatz, der für den zweiten Teilbereich ermittelt wurde, $\Delta f_0^{(2)}, \Delta G_x^{(2)}, \Delta G_y^{(2)}, \Delta G_z^{(2)}$ wird dabei vorteilhafterweise derart gewählt, dass lokale Feldabweichungen $AB_0(x,y,z)$ im zweiten Teilbereich optimal kompensiert werden.

Zum Ermitteln der Shimparametersätze für den ersten und/oder zweiten Teilbereich kann für jeden Pixel der ersten B0-Feldkarte innerhalb des ersten Teilbereiches und/oder des zweiten Teilbereiches eine Gleichung der folgenden Form aufgestellt werden:

$$-\Delta B_o(x,y,z) = \frac{2\pi}{\gamma}\Delta f_0 + \Delta G_x(x-x_0) + \Delta G_y(y-y_0) + \Delta G_z(z-z_0)$$

Darin ist (x,y,z) die Koordinate des jeweiligen Pixels, (x0, y0, z0) die Koordinate eines Iso-Zentrums (isocentre) der Gradientenspuleneinheit 19 innerhalb der BO-Feldkarte. Das Isozentrum ist der Ort an dem der Feldbeitrag der Gradientenspuleneinheit 19 Null ist. $\Delta G_x$ gibt eine Änderung des Gradientenfeldes entlang der x-Richtung der BO-Feldkarte gegenüber der Einstellung während der Akquisition der BO-Feldkarte an. Entsprechend geben $\Delta G_y$ und $\Delta G_z$ eine Änderung des Gradientenfeldes entlang der y- bzw. z-Richtung der BO-Feldkarte gegenüber der entsprechenden Einstellung während der Akquisition der BO-Feldkarte an. $\Delta f_0$ gibt eine Änderung einer Mittenfrequenz gegenüber der Einstellung während der Akquisition der BO-Feldkarte an.

In Ihrer Gesamtheit bilden die erwähnten Gleichungen für jeden Teilbereich des Untersuchungsbereichs ein überbestimmtes lineares Gleichungssystem, das mit Standardmethoden gelöst werden kann. So können die jeweils vier unbekannten $\Delta f_0^{(1)}$, $\Delta G_x^{(1)}$, $\Delta G_y^{(1)}$, $\Delta G_z^{(1)}$ des ersten Shimparametersatzes für den ersten Teilberiech bzw. $\Delta f_0^{(2)}$, $\Delta G_x^{(2)}$, $\Delta G_y^{(2)}$, $\Delta G_z^{(2)}$ des ersten Shimparametersatzes für den zweiten Teilbereich ermittelt werden. Beruht die Erstellung der BO-Feldkarte auf der Messung der Phasendifferenz zwischen akquirierten MR-Bildern mit unterschiedlicher Echozeit, so kann aus Pixeln, die im Wesentlichen nur Rauschen enthalten, keine Information über die lokale B0-Feldabweichung gewonnen werden. Solche Pixel sind also bei der Lösung der Gleichungssysteme auszuschließen. Dies kann durch eine Hintergrundsegmentierung und/oder eine Gewichtung der einzelnen Gleichungen proportional zum Pixel-wert eines Betragsbildes am Ort (x,y,z) erfolgen. Das Betragsbild kann dabei ebenfalls aus den Magnetresonanz-Messdaten, die zur Erstellung der ersten BO-Feldkarte akquiriert werden extrahiert werden.

In einem weiteren Verfahrensschritt 44 erfolgt eine Berechnung einer zweiten BO-Feldkarte unter Verwendung der ersten BO-Feldkarte und der mehreren ersten Shimparametersätze. Die zweite BO-Feldkarte wird unter Verwendung der ersten B0-Felkarte und der mehreren ersten Shimparametersätze derart berechnet, dass B0-Feldbeiträge, welche sich aus den mehreren ersten Shimparametersätzen ergeben, mit der ersten B0-Feldkarte verrechnet werden. Hierbei wird insbesondere ein räumlicher Abschnitt der ersten BO-Feldkarte mit den B0-Feldbeiträgen, welche sich aus einem geeigneten ersten Shimparametersatz der mehreren ersten Shimparametersätze ergeben, verrechnet, wobei der geeignete erste Shimparametersatz spezifisch für den Teilbereich der mehreren Teilbereiche, welcher zu dem räumlichen Abschnitt korrespondiert, ermittelt wurde.

Im gezeigten Fall wird in einem ersten Teilschritt 44-1 des weiteren Verfahrensschritts 44 ein erster räumlicher Teilbereich der zweiten BO-Feldkarte anhand des ersten räumlichen Teilbereichs der ersten BO-Feldkarte und des für den ersten Teilbereich ermittelten ersten Shimparametersatzes berechnet. In einem zweiten Teilschritt 44-2 des weiteren Verfahrensschritts 44 wird ein zweiter räumlicher Teilbereich der zweiten BO-Feldkarte anhand des zweiten räumlichen Teilbereichs der ersten BO-Feldkarte und des für den zweiten Teilbereich ermittelten ersten-Shimparametersatzes berechnet. Die zweite BO-Feldkarte umfasst dann die zwei berechneten räumlichen Teilbereiche.

Zur Berechnung der zweiten BO-Feldkarte werden beispielsweise für jeden Teilbereich die BO-Feldbeiträge des für den jeweiligen Teilbereich ermittelten ersten Shimparametersatz für den zumindest einen ersten Shimkanal 36 zu der ersten B0-Feldkarte pixelweise addiert. Das heißt, dass die zweite B0-Feldkarte eine neue virtuelle BO-Feldkarte $\Delta B_0'(x,y,z)$ darstellt, für die gilt:

$$\Delta B'_0(x,y,z) = \Delta B_o(x,y,z) + \begin{cases} \dfrac{2\pi}{\gamma}\Delta f_0^{(1)} + \Delta G_x^{(1)}(x-x_0) + \Delta G_y^{(1)}(y-y_0) + \Delta G_z^{(1)}(z-z_0), if\ (x,y,z) \in V1 \\ \dfrac{2\pi}{\gamma}\Delta f_0^{(2)} + \Delta G_x^{(2)}(x-x_0) + \Delta G_y^{(2)}(y-y_0) + \Delta G_z^{(2)}(z-z_0), if\ (x,y,z) \in V2 \end{cases}$$

Darin bezeichnet V1 den ersten Teilbereich, V2 den zweiten Teilbereich und das Elementzeichen aus der Mengenlehre steht hier für "der betrachtete Pixel mit Koordinaten (x,y,z) gehört zum ersten Teilbereich V1 bzw. zum zweiten Teilbereich V2". Die zweite, insbesondere virtuelle, BO-Feldkarte $\Delta B_0'(x,y,z)$ wird im allgemeinen geringere B0-Feldabweichungen als die ursprüngliche gemessene erste BO-Feldkarte $\Delta B_0(x,y,z)$ aufweisen. Lokal innerhalb eines Teilbereichs kann die zweite BO-Feldkarte den B0-Feldverlauf nach Einstellung des zumindest einen ersten Shimkanals 37 anhand des jeweiligen ersten Shimparametersatzes näherungsweise widergeben.

In dem weiteren Verfahrensschritt 41, dem Ermitteln des zweiten Shimparametersatzes, werden die mehreren ersten Shimparametersätze bei dem Ermitteln des zweiten Shimparametersatzes derart berücksichtigt, dass der zweite Shimparametersatz unter Verwendung der zweiten BO-Feldkarte ermittelt wird. Dabei wird der zweite Shimparametersatz vorteilhafterweise derart ermittelt, dass das durch die zweite B0-Feldkarte $\Delta B_0'(x,y,z)$ widergegebene B0-Feld möglichst optimal kompensiert wird.

Der Feldbeitrag $\Delta B_j(x, y, z)$ des j-ten Shimkanals des zumindest einen zweiten Shimkanals 37 am Ort (x,y,z) ist proportional zum Shimstrom $I_j$ im j-ten Shimkanal:

$$\Delta B_j(x,y,z) = C_j(x,y,z) \bullet P_j \bullet I_j$$

$C_j(x,y,z)$ ist die bekannte normierte Feldverteilung des zweiten Shimkanals j und $P_j$ die ebenfalls bekannte ortsunabhängige Sensitivität des zweiten Shimkanals j. Der Index j nimmt Werte von 1, ..., N2 an, wobei N2 die Anzahl der zweiten Shimkanäle 37 ist. Zur Ermittlung der unbekannten Shimströme $I_1$, ..., $I_{N2}$ kann für jeden Pixel im Untersuchungsbereich eine Gleichung der folgenden Form aufgestellt werden:

$$-\Delta B'_o(x,y,z) = \frac{2\pi}{\gamma}\Delta f_0 + \sum_{j=1}^{N2}\Delta B_j(x,y,z) = \frac{2\pi}{\gamma}\Delta f_0 + \sum_{j=1}^{N2}C_j(x,y,z)\cdot P_j\cdot\Delta I_j$$

Mit Untersuchungsbereich ist hier das Vereinigungsvolumen der beiden Teilbereiche gemeint. In ihrer Gesamtheit bilden die genannten Gleichungen also wieder ein überbestimmtes lineares Gleichungssystem. Überbestimmt weil die Zahl der Pixel im Untersuchungsbereich im Allgemeinen sehr viel größer ist als die Zahl der Unbekannten. Die gesuchten Unbekannten in diesem Gleichungssystem sind die N2 Werte $\Delta I_j$, die die Änderung des Stroms im j-ten Shimkanal gegenüber der Einstellung während der Akquisition der ersten B0-Feldkarte angeben, sowie wiederum ein Term $\Delta f_0$ ohne Ortsabhängigkeit. Das überbestimmte Gleichungssystem kann beispielsweise im Sinne der kleinsten quadratischen Abweichung mit Standardmethoden gelöst werden.

Ergebnis des Schrittes 41 ist also ein zweiter Shimparametersatz $\Delta f_0, I_1, ..., I_{N2}$, zur Einstellung des zumindest einen zweiten Shimkanals 37.

Das Erfassen der Magnetresonanz-Bilddaten im weiteren Verfahrensschritt 42 erfolgt typischerweise in einer verschachtelten 2D-Mehrschichtmessung. Die Zahl der Schichten kann typischerweise zwischen 5 und 50 liegen. Jede dieser Schichten kann eine Dicke von typischerweise 2mm bis 10mm aufweisen. Das Gesichtsfeld (field of view) einer Schicht liegt typischerweise zwischen 200 x 200 mm$^2$ (beispielsweise bei einer axialen Kopfuntersuchung) und 400 x 400 mm$^2$ (beispielsweise bei einer abdominellen Untersuchung). Die Schichten sind in der Regel parallel zueinander orientiert mit einer Lücke von 0 bis 50 Prozent der Schichtdicke zwischen benachbarten Schichten. Jede dieser Schichten und/oder ein Teilbereich jeder Schicht kann einen Teilbereich des Untersuchungsbereichs darstellen. Im gezeigten Fall liegen der Übersichtlichkeit halber jedoch nur zwei Teilbereiche des Untersuchungsbereichs vor. Die Zeit zwischen der Akquisition von Daten verschiedener Schichten beträgt TR/N, wobei TR die Repetitionszeit ist und N die Zahl der Schichten von denen Daten in einem Repetitionsintervall akquiriert werden. Die Repetitionszeit TR gibt die Zeit zwischen aufeinanderfolgenden Anregungen einer bestimmten Schicht an. Die TR Zeit liegt je nach zugrundeliegender Sequenztechnik zwischen wenigen ms und mehreren Sekunden. Die Zeit zwischen der Akquisition von Daten verschiedener Schichten ist entsprechend um einen Faktor N verkürzt. Die hier beschriebenen Parameter sind selbstverständlich nur Beispiele. Das Erfassen der Magnetresonanz-Bilddaten kann im weiteren Verfahrensschritt 42 auch mit anderen Messparametern als den beschriebenen Messparametern erfolgen.

Das Erfassen der Magnetresonanz-Bilddaten im weiteren Verfahrensschritt 42 umfasst einen ersten Teilschritt 42-1, bei dem zunächst der zumindest eine zweite Shimkanal 37 anhand des zweiten Shimparametersatzes eingestellt wird. Beispielsweise werden die fünf zweiten Shimkanäle 37 anhand der Shimströme $I_1, ..., I_5$ eingestellt.

In einem zweiten Teilschritt 42-2 des weiteren Verfahrensschritts 42 vergeht zwischen dem Einstellen des zumindest einen zweiten Shimkanals 37 und dem Erfassen der Magnetresonanz-Bilddaten aus dem ersten Teilbereich in einem vierten Teilschritt 42-4 eine zweite Einschwingzeit. Diese zweite Einschwingzeit ist dabei länger als eine erste Einschwingzeit, welche zwischen dem Einstellen des zumindest einen ersten Shimkanals 36 anhand des ersten Shimparametersatzes, der für den ersten Teilbereich ermittelt wurde, und dem Erfassen der Magnetresonanz-Bilddaten aus dem ersten Teilbereich, vergeht. Die Einschwingdauer wird abgewartet, bis der tatsächliche Feldbeitrag des zumindest einen zweiten Shimkanals 37 dem berechneten Feldbeitrag entspricht.

In einem dritten Teilschritt 42-3 des weiteren Verfahrensschritts 42 wird anschließend der zumindest eine erste Shimkanal 36 anhand des ersten Shimparametersatzes, der für den ersten Teilbereich ermittelt wurde, eingestellt. Im Beispiel werden also die Gradientenoffsetströme $\Delta G_x^{(1)}$, $\Delta G_y^{(1)}$, $\Delta G_z^{(1)}$ zu den Gradientenoffsetströmen, die während der Akquisition der ersten B0-Feldkarte eingestellt waren, addiert. Des Weiteren kann die RF-Mittenfrequenz um $\Delta f_0^{(1)} + \Delta f_0$ gegenüber dem Wert während der Akquisition der ersten B0-Feldkarte geändert werden. Dabei ist $\Delta f_0^{(1)}$ die im Schritt M3 ermittelte Mittenfrequenzänderung für den ersten Teilbereich und $\Delta f_0$ ist der konstante Term ohne Ortsabhängigkeit aus dem zweiten Shimparametersatz.

In einem vierten Teilschritt 42-4 des weiteren Verfahrensschritts 42 werden anschließend Magnetresonanz-Bilddaten aus dem ersten Teilbereich erfasst. Der zumindest eine erste Shimkanal 36 bleibt für das Erfassen der Magnetresonanz-Bilddaten aus dem ersten Teilbereich anhand des ersten Shimparametersatzes, der für den ersten Teilbereich ermittelt wurde, eingestellt.

In einem fünften Teilschritt 42-5 des weiteren Verfahrensschritts 42 wird anschließend der zumindest eine erste Shimkanal 36 anhand des ersten Shimparametersatzes, der für den zweiten Teilbereich ermittelt wurde, eingestellt. Dazu werden die Gradientenoffsetströme $\Delta G_x^{(2)}$, $\Delta G_y^{(2)}$, $\Delta G_z^{(2)}$ zu den Gradientenoffsetströmen, die während der Akquisition der ersten B0-Feldkarte eingestellt waren, addiert. Die Einstellung der RF-Mittenfrequenz wird gegenüber der Einstellung während der Akquisition der ersten B0-Feldkarte um $\Delta f_0^{(2)} + \Delta f_0$ geändert.

Es sei dabei angemerkt, dass bei der Einstellung des zumindest einen ersten Shimkanals 36 für einen bestimmten Teilbereich nur die Einstellungen zwischen Sende- und Empfangspfad der Magnetresonanz-Sequenz aktiv sein können. Ob beispielsweise die Gradientenoffsetströme zeitlich vor einem ersten Hochfrequenz-Anregungspuls innerhalb eines Teilbereichs eingeschaltet werden oder beispielsweise erst mit einem Schichtselektionsgradienten des ersten Hochfrequenz-Anregungspulses ist, sofern man physikalische Effekte in Folge des veränderten Gradientenstromes nicht berücksichtigt, irrelevant. Beispielsweise, kann es wegen unvermeidlichen Wirbelströmen (eddy currents) in Folge des veränderten Gradientenstromes, die mit einer typischen Zeitkonstante exponentiell abklingen, sinnvoll sein, den zumindest einen ersten Shimkanal 36 anhand der ersten Shimparametersätze so früh wie ohne Verlängerung der Gesamtmesszeit möglich einzustellen.

In einem sechsten Teilschritt 42-6 des weiteren Verfahrensschritts 42 werden anschließend Magnetresonanz-Bilddaten aus dem zweiten Teilbereich erfasst. Der zumindest eine erste Shimkanal 36 bleibt für das Erfassen der Magnetresonanz-Bilddaten aus dem zweiten Teilbereich anhand des ersten Shimparametersatzes, welcher für den zweiten Teilbereich ermittelt wurde, eingestellt.

Die Verfahrensschritte 42-3 bis 42-6 können so oft wiederholt werden, bis alle Magnetresonanzdaten aus den beiden Teilbereichen erfasst sind, die zur Rekonstruktion der Bilder nötig sind.

In einem siebten Teilschritt 42-7 des weiteren Verfahrensschritts 42 werden die Magnetresonanz-Bilddaten, welche den ersten Teilbereich abbilden, und die Magnetresonanz-Bilddaten, welche den zweiten Teilbereich abbilden, zu einem gesamten Magnetresonanz-Bilddatensatz zusammengeführt. Aus diesem gesamten Magnetresonanz-Bilddatensatz können dann Magnetresonanzbilder aus den beiden Teilbereichen rekonstruiert werden. Die Bilder können dann beispielsweise auf der Anzeigeeinheit 25 ausgegeben und/oder in einer Datenbank abgespeichert werden.

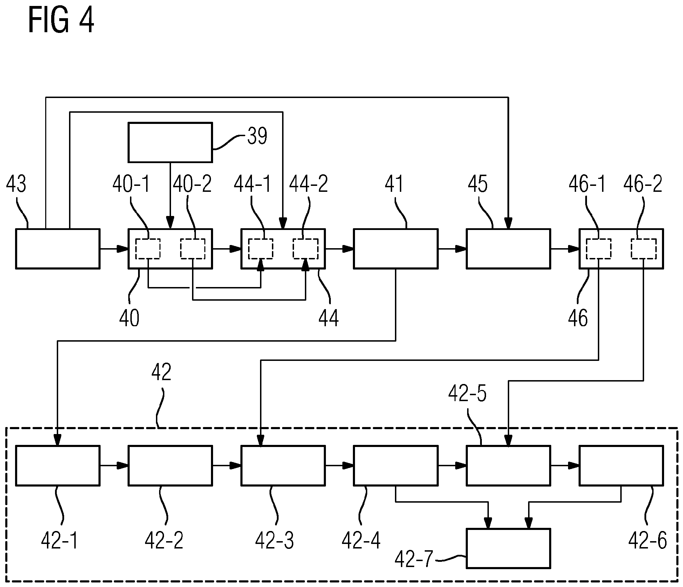

**Fig. 4** zeigt ein Ablaufdiagramm einer zweiten Ausführungsform eines erfindungsgemäßen Verfahrens zur Magnetresonanz-Bildgebung in einem Untersuchungsbereich eines Untersuchungsobjekts 15 mittels eines Magnetresonanzgeräts 11.

Die nachfolgende Beschreibung beschränkt sich im Wesentlichen auf die Unterschiede zu dem Ausführungsbeispiel in Fig. 3, wobei bezüglich gleich bleibender Verfahrensschritte auf die Beschreibung des Ausführungsbeispiels in Fig. 3 verwiesen wird. Im Wesentlichen gleich bleibende Verfahrensschritte sind grundsätzlich mit den gleichen Bezugszeichen beziffert.

Im Gegensatz zu dem in Fig. 3 gezeigten Vorgehen wird der zumindest eine erste Shimkanal 36 während des Erfassens der Magnetresonanz-Bilddaten im weiteren Verfahrensschritt 42 nicht anhand der im weiteren Verfahrensschritt 40 ermittelten mehreren ersten Shimparametersätze eingestellt.

Vielmehr wird gemäß Fig. 4 in einem weiteren Verfahrensschritt 45 eine dritte BO-Feldkarte unter Verwendung der ersten BO-Feldkarte und des zweiten Shimparametersatzes berechnet. Dabei kann Rechnung getragen werden, dass die modifizierten Shimströme in dem zumindest einen zweiten Shimkanal 37 das B0-Feld in den Teilbereichen des Untersuchungsbereichs beeinflussen. Bei der Berechnung der ersten Shimparametersätze des zumindest einen ersten Shimkanals 36 im weiteren Verfahrensschritt 40 wird dieser Beitrag zunächst nicht berücksichtigt. Deshalb werden die bekannten Feldbeiträge des zumindest einen zweiten Shimkanals 37 zu der ursprünglichen ersten B0-FeldKarte $\Delta B_0(x,y,z)$ addiert, wobei eine dritte B0-Feldkarte $\Delta B_0''(x, y, z)$ erzeugt wird:

$$\Delta B''_0(x, y, z) = \Delta B_o(x, y, z) + \frac{2\pi}{\gamma} \Delta f_0 + \sum_{j=1}^{N2} C_j(x, y, z) \cdot P_j \cdot \Delta I_j$$

Die neue dritte BO-Feldkarte $\Delta B_0''(x, y, z)$ gibt (bis auf den ortsunabhängigen Term $(2\pi/\gamma)\Delta f_0$) zumindest näherungsweise den Feldverlauf nach Einstellung des zumindest einen zweiten Shimkanals 37 wieder.

Anschließend erfolgt in einem weiteren Verfahrensschritt 46 ein Ermitteln von mehreren angepassten ersten Shimparametersätzen für den zumindest einen ersten Shimkanal 36 unter Verwendung der dritten BO-Feldkarte. So werden in einem ersten Teilschritt 46-1 des weiteren Verfahrensschritts 46 ein angepasster erster Shimparametersatz für den ersten Teilbereich und in einem zweiten Teilschritt 46-2 des weiteren Verfahrensschritts 46 ein angepasster zweiter Shimparametersatz für den zweiten Teilbereich ermittelt. Ergebnis ist ein neuer Satz von Shimeinstellungen $\Delta f_0'^{(1)}, \Delta G_x'^{(1)}, \Delta G_y'^{(1)}, \Delta G_z'^{(1)}$ für den ersten Teilbereich bzw. $\Delta f_0'^{(2)}, \Delta G_x'^{(2)}, \Delta G_y'^{(2)}, \Delta G_z'^{(2)}$ für den zweiten Teilbereich.

Während des Erfassens der Magnetresonanz-Bilddaten im weiteren Verfahrensschritt 42 wird der zumindest eine erste Shimkanal 36 anhand der mehreren angepassten ersten Shimparametersätze eingestellt. So wird im dritten Teilschritt 42-3 des weiteren Verfahrensschritts 42 der zumindest eine erste Shimkanal 36 anhand des angepassten ersten Shimparametersatzes für den ersten Teilbereich eingestellt. Im fünften Teilschritt 42-5 des weiteren Verfahrensschritts 42 wird anschließend der zumindest eine erste Shimkanal 37 anhand des angepassten zweiten Shimparametersatzes für den zweiten Teilbereich eingestellt.

Es sei erwähnt, dass die Ermittelung angepasster erster Shimparametersätze und angepasster zweiter Shimparametersätze auch iterativ erfolgen kann. Dazu werden in einem i-ten Iterationsschritt jeweils die Verfahrensschritte 45, 46, 44, 41 in der angegebenen Reihenfolge ausgeführt. Im Verfahrensschritt 45 wird dabei eine B0''(i)-Feldkarte unter Verwendung der ersten BO-Feldkarte und des zweiten Shimparametersatzes des vorangegangenen Iterationsschrittes berechnet. Im Verfahrensschritt 46 werden angepasste erste Shimparametersätze für die einzelnen Teilbereiche berechnet unter Verwendung der im jeweiligen Verfahrensschritt 45 berechneten BO-Feldkarte B0''(i). Im Verfahrensschritt 44 wird unter Verwendung der im jeweiligen Iterationsschritt berechneten B0"(i)-Feldkarte (Schritt 45) und den im jeweiligen im Verfahrensschritt 46 ermittelten angepassten ersten Shimparametersätzen für die einzelnen Teilbereiche eine weitere BO-Feldkarte B0'(i) berechnet. Im Verfahrensschritt 41 wird jeweils ein angepasster zweiter Shimparametersatz auf Basis der in Verfahrensschritt 41 des i-ten Iterationsschrittes berechneten B0-Feldkarte B0'(i) berechnet. Abbruchkriterium des iterativen Verfahrens ist, wenn die Änderung der angepassten ersten Shimparametersätze des i-ten Schrittes eines jeden Teilbereiches gegenüber dem angepassten ersten Shimparametersatz des (i-1)-ten Iterationsschrittes für den entsprechenden Teilbereich kleiner ist als ein für die ersten Shimkanäle vorgegebener erster Schwellwertsatz und die Änderung des im i-ten Iterationsschritt berechneten zweiten Shimparametersatzes gegenüber dem im (i-1)-ten Schritt berechneten zweiten Shimparametersatz kleiner ist wie ein für die zweiten Shimkanäle vorgegebener zweiter Schwellwertsatz oder wenn eine vorgegebene Maximalzahl von Iterationsschritten überschritten wird.

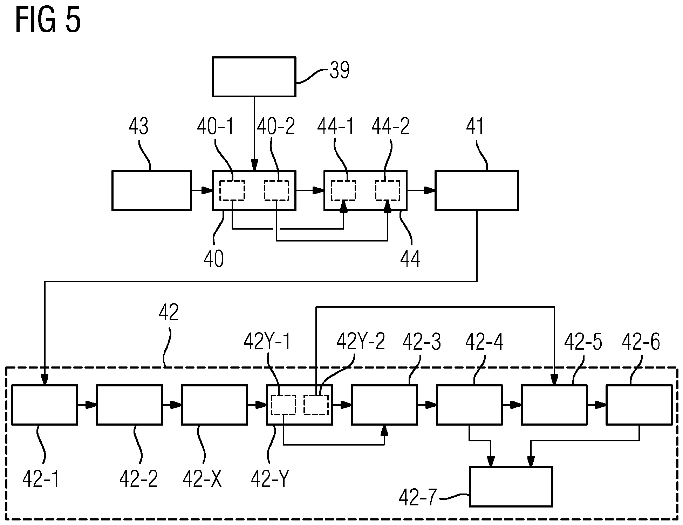

**Fig. 5** zeigt ein Ablaufdiagramm einer dritten Ausführungsform eines erfindungsgemäßen Verfahrens zur Magnetresonanz-Bildgebung in einem Untersuchungsbereich eines Untersuchungsobjekts 15 mittels eines Magnetresonanzgeräts 11.

Die nachfolgende Beschreibung beschränkt sich im Wesentlichen auf die Unterschiede zu dem Ausführungsbeispiel in Fig. 3, wobei bezüglich gleich bleibender Verfahrensschritte auf die Beschreibung des Ausführungsbeispiels in Fig. 3 verwiesen wird. Im Wesentlichen gleich bleibende Verfahrensschritte sind grundsätzlich mit den gleichen Bezugszeichen beziffert.

Im Gegensatz zu dem in Fig. 3 gezeigten Vorgehen wird der zumindest eine erste Shimkanal 36 während vor dem Erfassen der Magnetresonanz-Bilddaten aus einem Teilbereich im weiteren Verfahrensschritt 42 nicht anhand des im weiteren Verfahrensschritt 40 ermittelten ersten Shimparametersatzes für den Teilbereich eingestellt. Auch diese Ausführungsform berücksichtigt bei der Berechnung der ersten Shimparametersätze für den zumindest einen ersten Shimkanal 36 den Einfluss des zumindest einen zweiten Shimkanals 37. Im Gegensatz zu der zweiten Ausführungsform gemäß Fig. 4 wird der Feldverlauf nach Einstellung des zumindest einen zweiten Shimkanals 37 aber nicht berechnet, sondern tatsächlich gemessen.

Vielmehr erfolgt gemäß Fig. 5 in einem weiteren Teilschritt 42-X des weiteren Verfahrensschritts 42 ein Erfassen einer vierten BO-Feldkarte nach dem Einstellen des zumindest einen zweiten Shimkanals 37 im ersten Teilschritt 42-1 des weiteren Verfahrensschritts 42. Die vierte BO-Feldkarte wird insbesondere frühestens dann erfasst, wenn die zweite Einschwingzeit im weiteren Teilschritt 42-2 des weiteren Verfahrensschritts 42 vergangen ist.

Anschließend erfolgt in einem weiteren Teilschritt 42-Y des weiteren Verfahrensschritts 42 ein Ermitteln von mehreren veränderten ersten Shimparametersätzen für den zumindest einen ersten Shimkanal 36 unter Verwendung der vierten B0-Feldkarte. So werden in einem ersten Unterschritt 42Y-1 des Teilschritts 42-Y ein angepasster erster Shimparametersatz für den ersten Teilbereich und in einem zweiten Unterschritt 42Y-2 des Teilschritts 42-Y ein angepasster zweiter Shimparametersatz für den zweiten Teilbereich ermittelt. Ergebnis ist wieder eine Mittenfrequenz $\Delta f_0"^{(1)}$, und ein Satz von ersten Shimparametersätzen $\Delta G_x"^{(i)}$, $\Delta G_y"^{(i)}$, $\Delta G_z"^{(i)}$ für den zumindest einen ersten Shimkanal 36 für jeden Teilbereich.

Während des Erfassens der Magnetresonanz-Bilddaten im weiteren Verfahrensschritt 42 wird der zumindest eine erste Shimkanal 36 anhand der mehreren angepassten ersten Shimparametersätze eingestellt. So wird im dritten Teilschritt 42-3 des weiteren Verfahrensschritts 42 der zumindest eine erste Shimkanal 36 anhand des angepassten ersten Shimparametersatzes für den ersten Teilbereich eingestellt. Im fünften Teilschritt 42-5 des weiteren Verfahrensschritts 42 wird anschließend der zumindest eine erste Shimkanal 37 anhand des angepassten zweiten Shimparametersatzes für den zweiten Teilbereich eingestellt.

Das Erfassen der ersten BO-Feldkarte und/oder der vierten B0-Feldkarte kann unter Verwendung von Rohdaten erfolgen, welche aus mindestens drei Echosignalen bestehen, die jeweils nach einer Anregung des Untersuchungsbereichs akquiriert werden.

Die in Fig. 2-5 dargestellten Verfahrensschritte werden von der Recheneinheit ausgeführt. Hierzu umfasst die Recheneinheit erforderliche Software und/oder Computerprogramme, die in einer Speichereinheit der Recheneinheit gespeichert sind. Die Software und/oder Computerprogramme umfassen Programmmittel, die dazu ausgelegt sind, das jeweilige Verfahren auszuführen, wenn das Computerprogramm und/oder die Software in der Recheneinheit mittels einer Prozessoreinheit der Recheneinheit ausgeführt wird.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen, der durch die Ansprüche definiert wird.

**Patentansprüche**

**1.** Verfahren zur Magnetresonanz-Bildgebung in einem Untersuchungsbereich eines Untersuchungsobjekts (15) mittels eines Magnetresonanzgeräts (11), welches eine Shimeinheit (35) umfasst, wobei die Shimeinheit (35) eine erste Shimkanalmenge mit zumindest einem ersten Shimkanal (36) und eine zweite Shimkanalmenge mit zumindest einem zweiten Shimkanal (37) umfasst, umfassend die folgenden Verfahrensschritte:

- Einteilen des Untersuchungsbereichs in mehrere Teilbereiche,
- Ermitteln von mehreren ersten Shimparametersätzen für den zumindest einen ersten Shimkanal (36), wobei jeweils ein erster Shimparametersatz der mehreren ersten Shimparametersätze für jeden der mehreren Teilbereiche ermittelt wird, wobei vor dem Ermitteln der mehreren ersten Shimparametersätze eine erste B0-Feldkarte erfasst wird, wobei das Ermitteln der mehreren ersten Shimparametersätze unter Verwendung der ersten B0-Feldkarte erfolgt,
- Ermitteln von einem zweiten Shimparametersatz für den zumindest einen zweiten Shimkanal (37) unter Berücksichtigung der mehreren ersten ermittelten Shimparametersätze,
- Erfassen von Magnetresonanz-Bilddaten des Untersuchungsbereichs des Untersuchungsobjekts (15), wobei vor dem Erfassen der Magnetresonanz-Bilddaten der zumindest eine zweite Shimkanal (37) anhand des zweiten Shimparametersatzes eingestellt wird und der zumindest eine erste Shimkanal (36) für das Erfassen der Magnetresonanz-Bilddaten aus einem bestimmten Teilbereich der mehreren Teilbereiche anhand eines für den bestimmten Teilbereich ermittelten ersten Shimparametersatzes eingestellt wird, **dadurch gekennzeichnet, dass** eine zweite B0-Feldkarte unter Verwendung der ersten B0-Feldkarte und der mehreren ersten Shimparametersätze berechnet wird, und die mehreren ersten Shimparametersätze bei dem Ermitteln des zweiten Shimparametersatzes derart berücksichtigt werden, dass der zweite Shimparametersatz unter Verwendung der zweiten B0-Feldkarte ermittelt wird.

2. Verfahren nach Anspruch 1, wobei die erste Shimkanalmenge und die zweite Shimkanalmenge disjunkt sind.

3. Verfahren nach einem der vorhergehenden Ansprüche, wobei die zweite B0-Feldkarte unter Verwendung der ersten B0-Feldkarte und der mehreren ersten Shimparametersätze derart berechnet wird, dass B0-Feldbeiträge, welche sich aus den mehreren ersten Shimparametersätzen ergeben, mit der ersten B0-Feldkarte verrechnet werden.

4. Verfahren nach Anspruch 3, wobei ein räumlicher Abschnitt der ersten B0-Feldkarte mit den B0-Feldbeiträgen, welche sich aus einem geeigneten ersten Shimparametersatz der mehreren ersten Shimparametersätze ergeben, verrechnet wird, wobei der geeignete erste Shimparametersatz spezifisch für den Teilbereich der mehreren Teilbereiche, welcher zu dem räumlichen Abschnitt korrespondiert, ermittelt wurde.

5. Verfahren nach einem der vorhergehenden Ansprüche, wobei eine dritte B0-Feldkarte unter Verwendung der ersten B0-Feldkarte und des zweiten Shimparametersatzes berechnet wird, wobei ein Ermitteln von mehreren angepassten ersten Shimparametersätzen für den zumindest einen ersten Shimkanal (36) unter Verwendung der dritten B0-Feldkarte erfolgt, wobei für das Erfassen der Magnetresonanz-Bilddaten der zumindest eine erste Shimkanal (36) anhand der mehreren angepassten ersten Shimparametersätze eingestellt wird.

6. Verfahren nach einem der vorhergehenden Ansprüche, wobei für das Erfassen der Magnetresonanz-Bilddaten

- zunächst der zumindest eine zweite Shimkanal (37) anhand des zweiten Shimparametersatzes eingestellt wird,
- anschließend der zumindest eine erste Shimkanal (36) anhand eines ersten Shimparametersatzes, welcher für einen ersten Teilbereich der mehreren Teilbereiche ermittelt wird, eingestellt wird,
- anschließend Magnetresonanz-Bilddaten aus dem ersten Teilbereich erfasst werden,
- anschließend der zumindest eine erste Shimkanal (36) anhand eines ersten Shimparametersatzes, welcher für einen zweiten Teilbereich der mehreren Teilbereiche ermittelt wird, eingestellt wird,
- anschließend Magnetresonanz-Bilddaten aus dem zweiten Teilbereich erfasst werden.

7. Verfahren nach Anspruch 6, wobei zwischen dem Einstellen des zumindest einen zweiten Shimkanals (37) und dem Erfassen der Magnetresonanz-Bilddaten aus dem ersten Teilbereich eine zweite Einschwingzeit vergeht und zwischen dem Einstellen des zumindest einen ersten Shimkanals (36) anhand des ersten Shimparametersatzes und dem Erfassen der Magnetresonanz-Bilddaten aus dem ersten Teilbereich eine erste Einschwingzeit vergeht, wobei die erste Einschwingzeit kürzer als die zweite Einschwingzeit ist.

8. Verfahren nach einem der Ansprüche 6-7, wobei nach dem Einstellen des zumindest einen zweiten Shimkanals (37) eine vierte B0-Feldkarte erfasst wird, wobei ein Ermitteln von mehreren veränderten ersten Shimparametersätzen für den zumindest einen ersten Shimkanal (36) unter Verwendung der vierten B0-Feldkarte erfolgt, wobei

während des Erfassens der Magnetresonanz-Bilddaten der zumindest eine erste Shimkanal (36) anhand der mehreren veränderten ersten Shimparametersätze eingestellt wird.

9. Verfahren nach einem der vorhergehenden Ansprüche, wobei das Erfassen der ersten BO-Feldkarte unter Verwendung von Rohdaten erfolgt, welche aus mindestens drei Echosignalen bestehen, die jeweils nach einer Anregung des Untersuchungsbereichs akquiriert werden.

10. Magnetresonanzgerät (11), umfassend eine Shimeinheit (35), welche eine erste Shimkanalmenge mit zumindest einem ersten Shimkanal (36) und eine zweite Shimkanalmenge mit zumindest einem zweiten Shimkanal (37) umfasst, eine Bilddatenerfassungseinheit (32) und eine Recheneinheit (24) mit einer Einteilungseinheit (38), einer ersten Ermittlungseinheit (33) und einer zweiten Ermittlungseinheit (34), wobei das Magnetresonanzgerät (11) dazu ausgebildet ist, ein Verfahren nach einem der vorhergehenden Ansprüche auszuführen.

11. Computerprogrammprodukt, welches direkt in einen Speicher einer programmierbaren Recheneinheit (24) eines Magnetresonanzgeräts (11) ladbar ist, mit Programmcode-Mitteln, um ein Verfahren nach einem der Ansprüche 1-9 auszuführen, wenn das Computerprogrammprodukt in der Recheneinheit (24) des Magnetresonanzgeräts (11) nach Anspruch 10 ausgeführt wird.

## Claims

1. Method for magnetic resonance imaging in an examination region of an examination object (15) by means of a magnetic resonance device (11) which comprises a shim unit (35), wherein the shim unit (35) comprises a first shim channel volume having at least one first shim channel (36) and a second shim channel volume having at least one second shim channel (37), comprising the following method steps:

   - dividing the examination region into a plurality of sections,
   - ascertaining a plurality of first shim parameter sets for the at least one first shim channel (36), wherein one first shim parameter set respectively of the plurality of first shim parameter sets is ascertained for each of the plurality of sections, wherein, before ascertaining the plurality of first shim parameter sets, a first B0 field map is acquired, wherein the plurality of first shim parameter sets is ascertained using the first B0 field map,
   - ascertaining a second shim parameter set for the at least one second shim channel (37) by taking into account the plurality of first ascertained shim parameter sets,
   - acquiring magnetic resonance image data of the examination region of the examination object (15), wherein, before acquiring the magnetic resonance image data, the at least one second shim channel (37) is adjusted using the second shim parameter set and the at least one first shim channel (36) is adjusted for acquiring the magnetic resonance image data from a specific section of the plurality of sections using a first shim parameter set ascertained for the specific section,

   **characterised in that**
   a second B0 field map is calculated using the first B0 field map and the plurality of first shim parameter sets, and the plurality of first shim parameter sets is taken into account when ascertaining the second shim parameter set such that the second shim parameter set is ascertained using the second B0 field map.

2. Method according to claim 1, wherein the first shim channel volume and the second shim channel volume are disjunct.

3. Method according to one of the preceding claims, wherein the second B0 field map is calculated using the first B0 field map and the plurality of first shim parameter sets such that B0 field contributions, resulting from the plurality of first shim parameter sets, are offset against the first B0 field map.

4. Method according to claim 3, wherein a spatial portion of the first B0 field map is offset against the B0 field contributions resulting from an appropriate first shim parameter set of the plurality of first shim parameter sets, wherein the appropriate first shim parameter set has been specifically ascertained for the section of the plurality of sections which corresponds to the spatial portion.

5. Method according to one of the preceding claims, wherein a third B0 field map is calculated using the first B0 field map and the second shim parameter set, wherein a plurality of adjusted first shim parameter sets is ascertained for the at least one first shim channel (36) using the third B0 field map, wherein the at least one first shim channel (36) is adjusted using the plurality of adjusted first shim parameter sets for acquiring the magnetic resonance image data.

6. Method according to one of the preceding claims, wherein for acquiring the magnetic resonance image data

   - firstly the at least one second shim channel (37) is adjusted using the second shim parameter set,
   - then the at least one first shim channel (36) is adjusted using a first shim parameter set which is ascertained for a first section of the plurality of sections,
   - then magnetic resonance image data is acquired from the first section,
   - then the at least one first shim channel (36) is adjusted using a first shim parameter set, which is ascertained for a second section of the plurality of sections,
   - then magnetic resonance image data is acquired from the second section.

7. Method according to claim 6, wherein a second settling time elapses between adjustment of the at least one second shim channel (37) and acquiring the magnetic resonance image data from the first section, and a first settling time elapses between adjusting the at least one first shim channel (36) using the first shim parameter set and acquiring the magnetic resonance image data from the first section, wherein the first settling time is shorter than the second settling time.

8. Method according to one of claims 6-7, wherein, after adjusting the at least one second shim channel (37), a fourth B0 field map is acquired, wherein a plurality of altered first shim parameter sets is ascertained for the at least one first shim channel (36) using the fourth B0 field map, wherein, during acquisition of the magnetic resonance image data, the at least one first shim channel (36) is adjusted using the plurality of altered first shim parameter sets.

9. Method according to one of the preceding claims, wherein the first B0 field map is acquired using raw data comprising at least three echo signals which are each acquired following an excitation of the examination region.

10. Magnetic resonance device (11), comprising a shim unit (35), which comprises a first shim channel volume having at least one first shim channel (36) and a second shim channel volume having at least one second shim channel (37), an image data acquisition unit (32) and an arithmetic unit (24) having a division unit (38), a first ascertaining unit (33) and a second ascertaining unit (34), wherein the magnetic resonance device (11) is designed to carry out a method according to one of the preceding claims.

11. Computer program product, which can be loaded directly into a memory of a programmable arithmetic unit (24) of a magnetic resonance device (11), having program code means to carry out a method according to one of claims 1-9 when the computer program product is run in the arithmetic unit (24) of the magnetic resonance device (11) according to claim 10.

**Revendications**

1. Procédé d'imagerie par résonance magnétique dans une région à examiner d'un objet (15) à examiner au moyen d'un appareil (11) à résonance magnétique, lequel comporte une unité (35) shim, l'unité (35) shim comportant un premier ensemble de canal shim ayant au moins un premier canal (36) shim et un deuxième ensemble de canal shim ayant au moins un deuxième canal (37) shim, le procédé comportant les étapes suivantes :

   - on subdivise la région à examiner en plusieurs régions partielles,
   - on détermine une pluralité de premiers ensembles de paramètres shim pour le au moins un premier canal (36) shim, un premier ensemble de paramètres de canal shim parmi les plusieurs premiers ensembles de paramètres de canal shim étant déterminé pour chacune des plusieurs régions partielles, dans lequel avant la détermination des plusieurs premiers ensembles de paramètres shim, il est obtenu une première carte de champ B0, la détermination des plusieurs premiers ensembles de paramètres shim s'effectuant en utilisant la première carte de champ B0,
   - on détermine un deuxième ensemble de paramètres shim pour le au moins un deuxième canal (37) shim en prenant en compte les plusieurs premiers ensembles de paramètres shim déterminés,
   - on acquiert des données d'image par résonnance magnétique de la région à examiner de l'objet (15) à examiner, dans lequel, avant l'acquisition des données d'image par résonnance magnétique on règle le au moins un deuxième canal (37) shim à l'aide du deuxième ensemble de paramètres shim et on règle le au moins un premier canal (36) shim pour l'acquisition des données d'image par résonnance magnétique à partir d'une région partielle déterminée des plusieurs régions partielles à l'aide d'un premier ensemble de paramètres shim déterminé pour la région partielle déterminée, **caractérisé en ce que** on calcule une deuxième carte de champ B0 en utilisant la première carte de champ B0 et les plusieurs premiers ensembles de paramètres shim, et, lors de la détermination du deuxième ensemble de paramètres shim, ont prend en compte les plusieurs premiers ensembles de paramètres shim de manière à ce que le deuxième ensemble de paramètres shim soit déterminé en utilisant la deuxième carte de champ B0.

2. Procédé suivant la revendication 1, dans lequel le premier ensemble de canal shim et le deuxième ensemble de canal shim sont disjoints.

3. Procédé suivant l'une des revendications précédentes, dans lequel la deuxième carte de champ B0 est calculée en utilisant la première carte de champ B0 et les plusieurs premiers ensembles de paramètres shim de manière à ce que des valeurs de champ B0, qui sont obtenues à partir des plusieurs premiers ensembles de paramètres shim, soient calculées avec la première carte de champ B0.

4. Procédé suivant la revendication 3, dans lequel une section spatiale de la première carte de champ B0 est calculée avec les valeurs de champ B0 qui sont obtenues à partir d'un premier ensemble de paramètres shim appropriés des plusieurs premiers ensembles de paramètres shim, le premier ensemble de paramètres shim appropriés étant déterminé de manière spécifique pour la région partielle des plusieurs régions partielles, qui correspond à la section spatiale.

5. Procédé suivant l'une des revendications précédentes, dans lequel une troisièmes carte de champ B0 est calculée en utilisant la première carte de champ B0 et le deuxième ensemble de paramètres shim, une détermination de plusieurs premiers ensembles de paramètres shim adaptés pour le au moins un premier canal (36) shim s'effectuant en utilisant la troisième carte de champ B0, pour l'acquisition des données d'image par résonnance magnétique, le au moins un premier canal (36) shim étant réglé à l'aide des plusieurs premiers ensembles de paramètres shim adaptés.

6. Procédé suivant l'une des revendications précédentes, dans lequel pour l'acquisition des données d'image par résonnance magnétique

   - il est d'abord réglé le au moins un deuxième canal (37) shim à l'aide du deuxième ensemble de paramètres shim,
   - ensuite le au moins un premier canal (36) shim est réglé à l'aide d'un premier ensemble de paramètres shim, qui est déterminé pour une première région partielle des plusieurs régions partielles,
   - ensuite des données d'image par résonnance magnétique sont acquises à partir de la première région partielle,
   - ensuite le au moins un premier canal (36) shim est réglé à l'aide d'un premier ensemble de paramètres shim, qui est déterminé pour une deuxième région partielle des plusieurs régions partielles,
   - ensuite des données d'image par résonnance magnétique sont acquises à partir de la deuxième région partielle.

7. Procédé suivant la revendication 6, dans lequel une deuxième durée transitoire s'écoule entre le réglage du au moins un deuxième canal (37) shim et l'acquisition des données d'image par résonnance magnétique à partir de la première région partielle et une première durée transitoire s'écoule entre le réglage du au moins un premier canal (36) shim à l'aide du premier ensemble de paramètres shim et l'acquisition des données d'image par résonnance magnétique à partir de la première région partielle, la première durée transitoire étant plus courte que la deuxième durée transitoire.

8. Procédé suivant l'une des revendications 6 à 7, dans lequel après le réglage du au moins un deuxième canal (37) shim, il est acquis une quatrième carte de champ B0, une détermination de plusieurs premiers ensembles de paramètres shim modifiés s'effectuant pour le au moins un premier canal (36) shim en utilisant la quatrième de champ B0, dans lequel, pendant l'acquisition des données d'image par résonnance magnétique, le au moins un premier canal (36) shim est réglé à l'aide des plusieurs premiers ensembles de paramètres shim modifiés.

9. Procédé suivant l'une des revendications précédentes, dans lequel l'acquisition de la première carte de champ B0 s'effectue par utilisation de données brutes, qui sont constituées d'au moins trois signaux d'écho, qui sont respectivement acquis à la suite d'une excitation de la région à examiner.

10. Appareil (11) de résonnance magnétique, comportant une unité (35) shim, laquelle comporte un premier ensemble de canal shim comportant au moins un premier canal (36) shim et un deuxième ensemble de canal shim comportant au moins un deuxième canal (37) shim, une unité (32) d'acquisition de données d'image et une unité (24) de calcul comportant une unité (38) de subdivision, une première unité (33) de détermination et une deuxième unité (34) de détermination, l'appareil (11) à résonnance magnétique étant agencé de manière à mettre en œuvre un procédé suivant l'une des revendications précédentes.

11. Produit de programme d'ordinateur, qui peut être chargé directement dans une mémoire d'une unité (24) informatique pouvant être programmée d'un appareil (11) à résonnance magnétique, comportant des moyens de codes de programme, afin de mettre en œuvre un procédé suivant l'une des revendications 1 à 9, lorsque le produit de programme d'ordinateur est mis en œuvre dans l'unité (24) informatique de l'appareil (11) à résonnance magnétique suivant la revendication 10.

# FIG 1

# FIG 2

# FIG 3

FIG 4

# FIG 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20080088307 A1

**In der Beschreibung aufgeführte Nicht-Patentliteratur**

- **SCHRIFT BLAMIRE et al.** Dynamic Shim Updating: A New Approach Towards Optimized Whole Brain Shimming. *MRM,* 1996, vol. 36, 159-165
- **SCHRIFT MORRELL et al.** Dynamic Shimming for Multi-Slice Magnetic Resonance Imaging. *MRM,* 1997, vol. 38, 477-483
- **SCHRIFTEN VON SENGUPTA et al.** Dynamic B0 shimming at 7T. *Magnetic Resonance Imaging,* 2011, vol. 29, 483-496
- **GRAAF et al.** Dynamic Shim Updating (DSU) for Multislice Signal Acquisition. *Magnetic Resonance in Medicine,* 2003, vol. 49, 409-416